# EUROPÄISCHE PATENTSCHRIFT

(11) **EP 3 479 860 B1**
(45) Veröffentlichungstag und Bekanntmachung des Hinweises auf die Patenterteilung: **24.02.2021**
(21) Anmeldenummer: 18000861.7
(22) Anmeldetag: 02.11.2018
(51) Int. Cl.: A61M 16/00, A61M 16/20

(54) **ATEMTHERAPIEGERÄT**
DEVICE FOR RESPIRATION THERAPY
DISPOSITIF DE THÉRAPIE RESPIRATOIRE

(30) Priorität: 03.11.2017 DE 102017010225
(43) Veröffentlichungstag der Anmeldung: 08.05.2019
(73) Patentinhaber: Löwenstein Medical Technology S.A., 2557 Luxembourg (LU)
(72) Erfinder: GÖBEL, Christof, 22457 Hamburg (DE)
(74) Vertreter: Marx, Thomas

(56) Entgegenhaltungen:
- WO-A2-2017/144963
- DE-A1- 19 914 749
- US-A1- 2014 290 659

## Beschreibung

Die vorliegende Erfindung betrifft ein Atemtherapiegerät, insbesondere Hustengerät, zur gezielten Unterstützung einer Sekretabfuhr aus den Atemwegen eines Patienten. Das Atemtherapiegerät umfasst wenigstens eine Strömungseinrichtung zum Erzeugen wenigstens einer Atemluftströmung für eine Insufflation in den Patienten und zum Erzeugen wenigstens einer Atemluftströmung für eine Exsufflation aus dem Patienten.

Bei bestimmten Erkrankungen kommt es zu einer direkten oder indirekten Beeinträchtigung der Sekretabfuhr aus den Atemwegen bzw. der Lunge. Um bei solchen Patienten eine Sekretabfuhr zu ermöglichen bzw. gezielt zu unterstützen, können sogenannte Hustengeräte bzw. Hustenmaschinen eingesetzt werden.

Bei gesunden Menschen kann das Sekret in der Regel durch Husten abgeführt werden. Bei dem Hustenvorgang erfolgt nach einem Einatmen eine plötzliche und kräftige Ausatembewegung. Dabei entsteht ein sehr schneller Luftstrom, mit dem das Sekret herausgeschleudert werden kann.

Die Hustengeräte können diesen Hustenvorgang durch eine gezielte Atemluftströmung unterstützen bzw. teilweise nachahmen. Dazu wird in der Regel zunächst Atemluft in die Lunge geblasen, die sog. Insufflation, und anschließend wieder abgesaugt, die sog. Exsufflation. Dabei sollte das Hustengerät in der Lage sein, die Exsufflation möglichst schnell und plötzlich auf die Insufflation folgen zu lassen. Weiterhin ist entscheidend, dass mit dem Hustengerät entsprechend hohe Strömungsgeschwindigkeiten für die Exsufflation erzeugt werden können.

Daher unterscheiden sich die Anforderungen an ein als Hustengerät ausgebildetes Atemtherapiegerät erheblich von den Anforderungen und Möglichkeiten, welche von Beatmungsgeräten bekannt sind.

Das in der US2014290659 (A1) beschriebene Hustengerät offenbart einen Aufbau, bei dem zwei Gasquellen einer Ventileinheit vorgeschaltet sind. Die Ventileinheit schaltet für die Exsufflation und Insufflation zwischen den Gasquellen um. Die Ventileinheit ist strömungstechnisch zwischen den Gasquellen und der Patientenschnittstelle, angeordnet.

Das in der DE19914749 (A1) beschriebene Beatmungsgerät offenbart ebenfalls einen Aufbau, bei dem zwei Gasquellen einer Ventileinheit vorgeschaltet sind. Die Ventileinheit ist strömungstechnisch zwischen den Gasquellen und der Patientenschnittstelle angeordnet.

Das in derWO2017144963 (A2) beschriebene Hustengerät offenbart einen Aufbau, bei dem je eine Gasquelle und ein Ventil in einem jeweils separaten pneumatischen Strömungsweg für die Exsufflation beziehungsweise für die Insufflation angeordnet sind. Der getrennte Aufbau für die Exsufflation beziehungsweise für die Insufflation soll Kontaminationen auf den Strömungsweg der Exsufflation begrenzen und so Infektionen des Patienten vorhindern.

Die bekannten Hustengeräte arbeiten in der Regel zuverlässig. Allerdings ist eine Verbesserung dieser Geräte von Vorteil, um den Patienten eine noch wirksamere bzw. angenehmere Unterstützung bei der Sekretabfuhr bieten zu können. Zudem ist der Einsatz der bekannten Hustengeräte oft mit entsprechend hohen Kosten verbunden.

Es ist daher die Aufgabe der vorliegenden Erfindung, ein Atemtherapiegerät zur Verfügung zu stellen, welches eine besonders wirksame und vorzugsweise auch angenehmere Unterstützung einer Sekretabfuhr aus den Atemwegen eines Patienten ermöglicht. Insbesondere soll auch eine besonders wirtschaftliche Herstellung des Atemtherapiegeräts möglich sein.

Diese Aufgabe wird gelöst durch ein Atemtherapiegerät mit den Merkmalen des Anspruchs 1. Bevorzugte Weiterbildungen sind Gegenstand der Unteransprüche. Weitere Vorteile und Merkmale der vorliegenden Erfindung ergeben sich aus der allgemeinen Beschreibung und der Beschreibung der Ausführungsbeispiele. Das erfindungsgemäße Atemtherapiegerät ist beispielsweise ein Hustengerät und dient zur gezielten Unterstützung einer Sekretabfuhr aus den Atemwegen eines Patienten mit wenigstens einer Strömungseinrichtung. Das erfindungsgemäße Atemtherapiegerät ist beispielsweise auch als ein kombiniertes Hustengerät und Beatmungsgerät oder als Beatmungsgerät ausgebildet. Die Strömungseinrichtung dient zum Erzeugen wenigstens einer Atemluftströmung für eine Insufflation in den Patienten und zum Erzeugen wenigstens einer Atemluftströmung für eine Exsufflation aus dem Patienten. Die Begriffe Insufflation und die Exsufflation werden im Sinne der vorliegenden Erfindung auch synonym für die Inspiration beziehungsweise Exspiration (im Rahmen der Beatmung) verwendet. Die Strömungseinrichtung umfasst wenigstens eine Patientenschnittstelle zum Verbinden des Patienten mit dem Atemtherapiegerät. Die Strömungseinrichtung umfasst wenigstens eine Atemluftschnittstelle zum Verbinden des Atemtherapiegeräts mit der Atemluft bzw. Umgebungsluft. Das Verbinden, ist im Sinne der Erfindung insbesondere als die Herstellung einer luftleitenden oder pneumatischen Verbindung zu verstehen. Dabei umfasst die Strömungseinrichtung wenigstens zwei parallel verlaufende Strömungswege mit jeweils wenigstens einer Ansaugseite und jeweils wenigstens einer Abgabeseite. Dabei umfasst die Strömungseinrichtung in den Strömungswegen jeweils wenigstens eine Gasquelle, beispielsweise als Lüfter ausgebildet, mit jeweils wenigstens einer Ansaugseite und jeweils wenigstens einer Abgabeseite. Wenigstens ein erster Lüfter ist mit seiner Ansaugseite und wenigstens ein zweiter Lüfter mit seiner Abgabeseite an wenigstens eine schaltbare Ventileinheit strömungstechnisch gekoppelt.

Die Gasquellen, beispielsweise die Lüfter, sind beispielsweise invers zueinander in den Strömungswegen angeordnet, wodurch sie beispielsweise einander entgegengesetzte Atemluftströmung erzeugen können. Die Gasquellen können beispielsweise als ein Lüfter und ein Ventil oder als zwei Lüfter oder als zwei Ventile ausgebildet sein. Erfindungsgemäß können auch eine Vielzahl an Gasquellen vorgesehen sein.

Erfindungsgemäß wird unter dem Begriff Insufflation insbesondere ein Belüften bzw. Befüllen der Lunge verstanden. Eine Insufflation umfasst insbesondere eine Luftzufuhr bzw. eine Inspiration im Rahmen eines Einatemvorgangs bzw. einer Beatmung.

Erfindungsgemäß wird unter dem Begriff Exsufflation insbesondere ein Entlüften der Lunge verstanden. Eine Exsufflation umfasst insbesondere eine Exspiration bzw. eine Luftabfuhr im Rahmen eines Ausatemvorgangs bzw. einer Beatmung. Das erfindungsgemäße Atemtherapiegerät bietet viele Vorteile. Ein erheblicher Vorteil ist, dass zwei parallel geschaltete Lüfter vorgesehen sind. Besonders vorteilhaft ist auch die schaltbare Ventileinheit, die mit den Lüftern entsprechend gekoppelt ist. Dadurch kann eine besonders zügige Umkehr des Volumenflusses bzw. Flows und/oder des Drucks verwirklicht werden.

So kann sehr kurzfristig zwischen der Insufflation und der Exsufflation gewechselt werden. Zudem können dadurch die Insufflation und Exsufflation auch sehr individuell an die Bedürfnisse des Patienten angepasst werden. Dadurch sind die Insufflation und Exsufflation auch besonders zeitgenau und exakt einstellbar. Das ist z. B. auch bei einer besonders sanften Unterstützung von Vorteil. Daher bietet das erfindungsgemäße Atemtherapiegerät eine besonders wirksame und zugleich sehr angenehme Unterstützung für die Sekretabfuhr. Aufgrund der wenigstens zwei Lüfter kann erheblich kurzfristiger zwischen Insufflation und Exsufflation gewechselt werden, da nicht erst eine Drehzahlanpassung oder dergleichen abgewartet werden muss. Mit der Ventileinheit kann dann zügig von einem Lüfter auf den anderen geschaltet werden, während z. B. eine Drehzahl der Lüfter bereits im Vorlauf entsprechend eingestellt wurde.

Die schaltbare Ventileinheit bietet zudem den Vorteil, dass auf zwei separate Strömungswege für jeden der Lüfter verzichtet werden kann. Dadurch wird der konstruktive Aufwand optimiert, sodass das erfindungsgemäße Atemtherapiegerät kostengünstig herstellbar ist und eine wirtschaftliche Sekrettherapie ermöglicht.

Insbesondere umfassen die wenigstens zwei Lüfter wenigstens einen ersten Lüfter und wenigstens einen zweiten Lüfter. Insbesondere sind die beiden Lüfter unabhängig voneinander betreibbar und vorzugsweise unabhängig voneinander steuerbar. Im Rahmen der vorliegenden Erfindung wird unter einem Steuern bzw. einer Steuerung vorzugsweise auch ein Regeln bzw. eine Regelung verstanden. Die Strömungseinrichtung umfasst insbesondere wenigstens eine Steuereinrichtung zum Steuern der Lüfter und/oder der Ventileinheit. Im Rahmen der vorliegenden Erfindung wird unter einem Verbinden vorzugsweise auch ein strömungstechnisches Verbinden bzw. Strömungsverbinden verstanden.

Die Ventileinheit ist vorzugsweise dazu geeignet und ausgebildet, je nach Ventilstellung entweder den ersten Lüfter oder den zweiten Lüfter mit der Atemluftschnittstelle strömungstechnisch zu verbinden. Die Ventileinheit kann auch dazu geeignet und ausgebildet sein, je nach Ventilstellung entweder den ersten Lüfter oder den zweiten Lüfter mit der Patientenschnittstelle zu verbinden. So kann je nach Ventilstellung die Atemluftströmung für die Insufflation dem Patienten zugeführt bzw. für die Exsufflation von dem Patienten weggeführt werden.

Die Atemluftströmung für die Insufflation weist also insbesondere eine umgekehrte Strömungsrichtung als die Atemluftströmung für die Exsufflation auf. Insbesondere ist durch ein Umschalten der Ventileinheit die Strömungsrichtung der Atemluftströmung dadurch umkehrbar, dass entweder der erste oder der zweite Lüfter zugeschaltet wird.

Vorzugsweise ist die Ventileinheit strömungstechnisch zwischen den wenigstens zwei Lüftern und der Atemluftschnittstelle angeordnet. Die Ventileinheit kann auch strömungstechnisch zwischen den wenigstens zwei Lüftern und der Patientenschnittstelle angeordnet sein. Solche Positionen für die Ventileinheit ermöglichen einen besonders zügigen Wechsel zwischen Insufflation und Exsufflation und sind konstruktiv besonders unaufwendig realisierbar.

Vorzugsweise sind die wenigstens zwei Lüfter gleichzeitig mit der Patientenschnittstelle bzw. dem Patienten strömungsverbunden, wenn die Ventileinheit strömungstechnisch zwischen den wenigstens zwei Lüftern und der Atemluftschnittstelle angeordnet ist. Vorzugsweise wird in Abhängigkeit einer Ventilstellung entweder der erste Lüfter oder der zweite Lüfter mit der Atemluftschnittstelle verbunden, wenn die Ventileinheit zwischen den Lüftern und der Atemluftschnittstelle angeordnet ist. Das hat den Vorteil, dass für den Wechsel zwischen Insufflation und Exsufflation nur die Atemluftschnittstelle mit dem entsprechenden Lüfter verbunden werden muss.

Der jeweils nicht mit der Atemluftschnittstelle verbundene Lüfter wird zum Patienten hin im Sinne der Luftförderung und Aufbau von Über-/ respektive Unterdruck in der jeweiligen Phase insbesondere nicht wirksam, da die Zuströmung zu diesem Lüfter bzw. Abströmung von diesem Lüfter durch die Ventileinheit vollständig oder zumindest weitgehend unterbunden wird. Vorzugsweise sind die wenigstens zwei Lüfter gleichzeitig mit der Atemluftschnittstelle strömungsverbunden, wenn die Ventileinheit strömungstechnisch zwischen den wenigstens zwei Lüftern und der Patientenschnittstelle angeordnet ist. Vorzugsweise wird in Abhängigkeit einer Ventilstellung entweder der erste Lüfter oder der zweite Lüfter mit der Patientenschnittstelle verbunden, wenn die Ventileinheit zwischen den wenigstens zwei Lüftern und der Patientenschnittstelle angeordnet ist. Das hat den Vorteil, dass für den Wechsel zwischen Insufflation und Exsufflation nur die Patientenschnittstelle mit dem entsprechenden Lüfter verbunden werden muss.

Das erfindungsgemäße Atemtherapiegerät dient zur Unterstützung einer Sekretabfuhr aus den Atemwegen eines Patienten mit wenigstens einer Strömungseinrichtung zum Erzeugen wenigstens einer Atemluftströmung für eine Insufflation in den Patienten und zum Erzeugen wenigstens einer Atemluftströmung für eine Exsufflation aus dem Patienten, wobei die Strömungseinrichtung wenigstens eine Patientenschnittstelle zum Verbinden des Patienten mit dem Atemtherapiegerät und wenigstens eine Atemluftschnittstelle zum Verbinden des Atemtherapiegeräts mit der Atemluft oder Umgebungsluft umfasst, wobei die Strömungseinrichtung wenigstens zwei Strömungswege aufweist, wobei jeder Strömungsweg wenigstens eine Gasquelle mit jeweils wenigstens einer Ansaugseite und jeweils wenigstens einer Abgabeseite umfasst und wobei die Gasquellen an wenigstens eine schaltbare Ventileinheit strömungstechnisch gekoppelt ist.

Die Ventileinheit ist vorzugsweise dazu geeignet und ausgebildet, in wenigstens einer ersten Ventilstellung die Ansaugseite des ersten Lüfters mit der Atemluftschnittstelle oder mit der Patientenschnittstelle zu verbinden und die Abgabeseite des zweiten Lüfters zu sperren.

Bevorzugt ist die Ventileinheit dazu geeignet und ausgebildet, in der ersten Ventilstellung die Ansaugseite des ersten Lüfters mit der Atemluftschnittstelle zu verbinden, wenn die Ventileinheit strömungstechnisch zwischen den Lüftern und der Atemluftschnittstelle angeordnet ist. Dabei wird insbesondere mit dem ersten Lüfter Atemluft angesaugt und in den Patienten geblasen. Die Ventileinheit kann auch dazu geeignet und ausgebildet sein, in der ersten Ventilstellung die Ansaugseite des ersten Lüfters mit der Patientenschnittstelle zu verbinden, wenn die Ventileinheit strömungstechnisch zwischen den Lüftern und der Patientenschnittstelle angeordnet ist. Dabei wird insbesondere mit dem ersten Lüfter Luft aus dem Patienten gesaugt und aus dem Gerät geblasen.

Bevorzugt ist die Ventileinheit dazu geeignet und ausgebildet, in wenigstens einer zweiten Ventilstellung die Ansaugseite des ersten Lüfters zu sperren und die Abgabeseite des zweiten Lüfters mit der Atemluftschnittstelle oder mit der Patientenschnittstelle zu verbinden.

Die Ventileinheit ist vorzugsweise dazu geeignet und ausgebildet, in der zweiten Ventilstellung die Abgabeseite des zweiten Lüfters mit der Atemluftschnittstelle zu verbinden, wenn die Ventileinheit strömungstechnisch zwischen den Lüftern und der Atemluftschnittstelle angeordnet ist. Dabei wird insbesondere mit dem zweiten Lüfter Luft aus dem Patienten gesaugt und aus dem Gerät geblasen. Die Ventileinheit kann auch dazu geeignet und ausgebildet sein, in der zweiten Ventilstellung die Abgabeseite des zweiten Lüfters mit der Patientenschnittstelle zu verbinden, wenn die Ventileinheit strömungstechnisch zwischen den Lüftern und der Patientenschnittstelle angeordnet ist. Dabei wird insbesondere mit dem zweiten Lüfter Atemluft angesaugt und zur Patientenschnittstelle bzw. in den Patienten geblasen.

Die Ventileinheit ist insbesondere dazu geeignet und ausgebildet, in wenigstens einer dritten Ventilstellung die Ansaugseite des ersten Lüfters zu sperren und die Abgabeseite des zweiten Lüfters zu sperren. In der dritten Ventilstellung ist insbesondere keiner der beiden Lüfter mit der Atemluftschnittstelle strömungsverbunden, wenn die Ventileinheit zwischen den Lüftern und der Atemluftschnittstelle angeordnet ist. In der dritten Ventilstellung ist die Patientenschnittstelle insbesondere mit keinem der beiden Lüfter strömungsverbunden, wenn die Ventileinheit zwischen den Lüftern und der Patientenschnittstelle angeordnet ist.

Solche Ventilstellungen bieten eine konstruktiv besonders unaufwendige Möglichkeit, um sehr zügig zwischen Insufflation und Exsufflation wechseln zu können. Bei allen Ventilstellungen sind Leckagen möglich und können beabsichtigt sein. Insbesondere ist in der ersten und/oder zweiten und/oder dritten Ventilstellung wenigstens eine Zwischenstellung einstellbar. Insbesondere umfasst eine Ventilstellung verschiedene Öffnungsgrade. Die Zwischenstellung umfasst insbesondere einen Öffnungsgrad, welcher zwischen vollständig geöffnet und vollständig geschlossen liegt. Insbesondere kann wenigstens ein Anschluss der Ventileinheit in der ersten und/oder zweiten und/oder dritten Ventilstellung vollständig geöffnet und vollständig geschlossen und vorzugsweise auch in einen dazwischen liegenden Öffnungsgrad gebracht werden.

In einer besonders bevorzugten Ausgestaltung umfasst die Ventileinheit wenigstens 3/3-Wegeventil. Ein solches Ventil arbeitet besonders zuverlässig und verlässlich und bietet eine besonders wirtschaftliche Ausgestaltung. Vorzugsweise umfasst das Wegeventil wenigstens drei Anschlüsse. Insbesondere umfasst das Wegeventil wenigstens drei Ventilstellungen. Besonders bevorzugt umfasst das Wegeventil die erste und die zweite und die dritte Ventilstellung. Insbesondere umfasst das Wegeventil wenigstens einen Anschluss für die Ansaugseite des ersten Lüfters und wenigstens einen Anschluss für die Abgabeseite des zweiten Lüfters und wenigstens einen Anschluss für die Atemluftschnittstelle oder Patientenschnittstelle. Die Ventileinheit kann auch ein Wegeventil mit mehr Ventilstellungen und/oder mehr Anschlüssen umfassen. Möglich sind auch weniger Ventilstellungen und/oder weniger Anschlüsse. Vorzugsweise ist die Ventileinheit als ein Proportionalventil ausgebildet oder umfasst wenigstens ein solches. Ein Proportionalventil ermöglicht eine besonders gezielte Anpassung der Insufflation bzw. Exsufflation an die Bedürfnisse des Patienten. Es bietet beispielsweise sehr zügige und zugleich besonders angenehme Übergänge. Die Ventileinheit kann auch ein Ventil mit diskreten Ventilstellungen umfassen oder als ein solches ausgebildet sein.

Besonders bevorzugt ist die Ventileinheit als ein 3/3-Wege-Proportionalventil ausgebildet. Das Proportionalventil ist insbesondere ein Ventil, bei dem ein Ergebnis an einem Anschluss bzw. Ausgang proportional zu einem Wert an einem anderen Anschluss bzw. am Eingang verändert werden kann. Insbesondere kann das Ergebnis am ersten und/oder zweiten Anschluss proportional zum Wert am dritten Anschluss verändert werden. Ob das Ergebnis am ersten oder zweiten Anschluss veränderbar ist, ist vorzugsweise davon abhängig, ob die erste oder zweite Ventilstellung eingenommen ist bzw. ob der erste oder zweite Anschluss mit dem dritten Anschluss verbunden ist. Insbesondere umfasst das Proportionalventil wenigstens drei Anschlüsse. Insbesondere ist bei einem wenigstens teilweise geöffneten ersten Anschluss ein zweiter Anschluss stets geschlossen. Insbesondere ist bei einem wenigstens teilweise geöffneten zweiten Anschluss der erste Anschluss stets geschlossen. Das hat den Vorteil, dass die Durchflussmenge an dem einen Anschluss einstellbar ist, während der Durchfluss an dem anderen Anschluss vollständig versperrt werden kann. Dabei sind Leckagen möglich und können beabsichtigt sein. Der erste Anschluss und der zweite Anschluss sind vorzugsweise mit jeweils einem Lüfter verbunden. Ein dritter Anschluss dient vorzugsweise als Eingang bzw. Ausgang für die anderen Anschlüsse. Der dritte Anschluss ist vorzugsweise immer offen. Der dritte Anschluss kann auch verschließbar bzw. öffenbar ausgebildet sein. Der dritte Anschluss ist vorzugsweise mit der Patientenschnittstelle oder mit der Atemschnittstelle verbunden.

Möglich und bevorzugt ist auch, dass bei einem wenigstens teilweise geöffneten ersten Anschluss ein zweiter Anschluss wenigstens teilweise geöffnet ist und/oder umgekehrt. Es ist möglich, dass die Öffnung an einem Anschluss in eine Öffnung an dem anderen Anschluss übergeht. Das Ventil kann dazu beispielsweise als Drehschieberventil ausgestaltet sein. Dann ist das Drehschieberventil z. B. derart ausgebildet, dass sich für die eine Drehrichtung das eine und für die andere Drehrichtung das andere Verhalten ergibt. In einem solchen Fall sind wenigstens zwei Öffnungen des Drehschieberventils auf dem Umfang des Ventilkörpers in ihren Winkellagen nicht gleich verteilt.

Vorzugsweise ist das Proportionalventil dazu geeignet und ausgebildet, einen Durchfluss für die Atemluftströmung für die Insufflation auf ein Maß zwischen einem maximalen Durchfluss und einem versperrten Durchfluss einzustellen und zugleich einen Durchfluss für die Atemluftströmung für die Exsufflation zu versperren. Möglich ist auch, dass das Proportionalventil dazu geeignet und ausgebildet ist, einen Durchfluss für die Atemluftströmung für die Exsufflation auf ein Maß zwischen einem maximalen Durchfluss und einem versperrten Durchfluss einzustellen und zugleich einen Durchfluss für die Atemluftströmung für die Insufflation zu versperren. Beispielsweise kann so eine definierte Ventilöffnung für die Ansaugseite des ersten Lüfters eingestellt werden, während die Abgabeseite des zweiten Lüfters versperrt ist.

Es ist bevorzugt, dass die Ventileinheit wenigstens ein Drehschieberventil umfasst oder als ein solches ausgebildet ist. Ein Drehschieberventil kann die erforderlichen Ventilstellungen besonders vorteilhaft umsetzen, da es zuverlässig arbeitet, sehr genaue angesteuert werden kann und wirtschaftlich ist. Das Drehschieberventil ist insbesondere als 3/3-Wegeventil ausgebildet. Das Drehschieberventil ist insbesondere als ein Proportionalventil ausgebildet. Das Drehschieberventil stellt insbesondere das 3/3-Wegeventil und/oder das Proportionalventil bereit. Das Drehschieberventil wird insbesondere von einem Schrittmotor angetrieben. Insbesondere sind die Ventilstellungen durch jeweils wenigstens eine axiale Drehung eines Ventilkolbens umsetzbar. Insbesondere sind die Anschlüsse für die Lüfter radial und/oder quer zur Drehachse des Ventilkolbens angeordnet. Insbesondere ist ein Anschluss für die Atemluftschnittstelle und/oder für die Patientenschnittstelle axial und/oder parallel zur Drehachse des Ventilkolbens angeordnet. Möglich sind auch andere Anordnungen der Anschlüsse. Die Anschlüsse für die Lüfter liegen sich insbesondere gegenüber. Die Anschlüsse der Lüfter können auch in einem anderen geeigneten Winkeln zueinander angeordnet sein. Insbesondere ist wenigstens eine Antriebseinheit zum Drehen des Ventilkolbens axial bzw. parallel zur Drehachse des Ventilkolbens angeordnet. Die Antriebseinheit kann auch radial bzw. quer zur Drehachse oder in einer anderen geeigneten Position zur Ventileinheit angeordnet sein.

Der Ventilkolben weist insbesondere wenigstens einen Kanal auf, welcher durch eine Drehung des Ventilkolbens mit wenigstens einem der Anschlüsse des Drehschieberventils verbindbar ist. Der Kanal umfasst insbesondere wenigstens zwei Kanalöffnungen, wobei wenigstens eine Kanalöffnung radial und wenigstens eine Kanalöffnung axial an dem Ventilkolben angeordnet ist. Möglich sind auch andere Anordnungen. Es ist möglich, dass der Kanal zu einem gegebenen Zeitpunkt nur mit jeweils einem der radialen Anschlüsse verbindbar ist. Es kann auch vorgesehen sein, dass der Kanal mit wenigstens zwei radialen Anschlüssen gleichzeitig verbindbar ist. Beispielsweise kann vorgesehen sein, dass die Öffnung zwei Anschlüsse gleichzeitig teilweise überdeckt.

Es ist möglich, dass die Anschlüsse des Drehschieberventils in Bezug auf einen Umfang des Ventilkolbens asymmetrisch bzw. nicht gegenüberliegend verteilt sind. Dabei kann der Ventilkolben vorzugsweise in zwei Drehrichtungen bewegt werden. Insbesondere kann der Kanal des Ventilkolbens dann in die eine Drehrichtung über einen ersten, längeren Weg und in die andere Drehrichtung über einen zweiten, kürzeren Weg mit dem Anschluss verbunden werden. Das hat den Vorteil, dass sich für die eine Drehrichtung ein anderes Verhalten als für die andere Drehrichtung ergibt. So kann eine konstruktiv unaufwendige und zugleich sehr wirksame Einstellung der Strömungsdynamik bzw. der Dynamik der Atemtherapie erfolgen.

Vorzugsweise erfolgt über den ersten Weg ein vollständiges und insbesondere längeres Verschließen des Anschlusses. Es ist möglich, dass der Anschluss über den zweiten Weg teilweise geöffnet bleibt oder dass über den zweiten Weg ein kürzeres Verschließen des Anschlusses erfolgt. Beispielsweise sind dem ersten Weg ein Drehwinkel von 270° und dem zweiten Weg ein Drehwinkel von 90° zugeordnet. Möglich sind auch andere Drehwinkel.

Es ist aber auch möglich und bevorzugt, dass die Anschlüsse des Drehschieberventils in Bezug auf einen Umfang des Ventilkolbens symmetrisch bzw. gegenüberliegend verteilt sind.

Die Ventileinheit kann auch wenigstens ein Ventil mit verschiebbaren Kolben und/oder eine andere geeignete umschaltbare Ventilbauweise umfassen.

In einer bevorzugten Weiterbildung ist die Strömungseinrichtung dazu geeignet und ausgebildet, wenigstens einen der wenigstens zwei Lüfter auch dann zu betreiben, wenn die Ansaugseite oder Abgabeseite des Lüfters durch die Ventileinheit gesperrt ist. Insbesondere ist wenigstens einer der wenigstens zwei Lüfter unabhängig von der Ventilstellung betreibbar. Dadurch können die Vorteile der Kombination aus wenigstens zwei Lüftern und wenigstens einer Ventileinheit besonders gut genutzt werden. Beispielsweise kann ein Lüfter aktiviert und eingestellt sein, bevor er durch die Ventileinheit zugeschaltet wird oder nachdem er durch die Ventileinheit gesperrt wurde.

Besonders bevorzugt ist die Strömungseinrichtung dazu geeignet und ausgebildet, einen geforderten Betriebspunkt wenigstens eines der wenigstens zwei Lüfter einzustellen, während die Ansaugseite oder Abgabeseite des einzustellenden Lüfters durch die Ventileinheit gesperrt ist. Dadurch hat der Lüfter die geforderte Drehzahl bereits erreicht, wenn er zugeschaltet wird. So ist ein besonders zügiger Wechsel von Insufflation und Exsufflation möglich und das Husten kann besonders wirksam unterstützt werden. Der Betriebspunkt definiert insbesondere eine Drehzahl und/oder eine Spannung und/oder wenigstens einen anderen charakteristischen Parameter für eine Einstellung des Lüfters. Die Einstellung umfasst beispielsweise ein Senken und/oder Erhöhen der Drehzahl.

Es kann auch vorgesehen sein, dass die Strömungseinrichtung dazu geeignet und ausgebildet ist, den geforderten Betriebspunkt und z. B. eine geforderte Drehzahl und/oder Spannung wenigstens eines der wenigstens zwei Lüfter einzustellen, wenn die Ventileinheit den einzustellenden Lüfter bereits mit der Atemluftschnittstelle oder Patientenschnittstelle verbunden hat. Das ermöglicht eine besonders gezielte Einstellung an die Bedürfnisse des Patienten.

Es ist möglich, dass das Atemtherapiegerät wenigstens eine Oszillatoreinrichtung umfasst. Die Oszillatoreinrichtung dient insbesondere zum Beaufschlagen der Atemluftströmung für die Insufflation und/oder Exsufflation mit wenigstens einer definierten Schwingung von wenigstens einer der Größen Flow und/oder Druck. Durch eine solche Oszillatoreinrichtung kann festsitzendes Sekret besonders gut gelöst werden.

Die definierte Schwingung ist vorzugsweise eine Druckschwingung und/oder eine Flussschwingung bzw. Flowschwingung der Atemluftströmung. Vorzugsweise ist die definierte Schwingung einstellbar. Vorzugsweise ist eine Frequenz und/oder eine Amplitude und/oder ein anderer charakteristischer Parameter zur Definition einer Schwingung einstellbar. Die Amplitude kann den Druck und/oder den Volumenfluss bzw. Flow der Atemluftströmung betreffen. Die Amplitude ist beispielsweise durch einen definierten Öffnungsgrad der Ventileinheit einstellbar. Die Frequenz ist beispielsweise durch eine Schaltfrequenz der Ventileinheit einstellbar. Es ist möglich, dass die Frequenz und/oder Amplitude für den Druck bzw. den Flow der Atemluftströmung einstellbar ist.

Die Oszillatoreinrichtung ist vorzugsweise dazu geeignet und ausgebildet, die Schwingung durch ein wiederholtes Umschalten der Ventileinheit zu erzeugen. So können Kosten und Bauteile eingespart werden, da die Ventileinheit bereits vorhanden ist. Insbesondere ist die Oszillatoreinrichtung dazu geeignet und ausgebildet, die Schwingung durch ein wenigstens teilweises Öffnen und ein wenigstens teilweises Schließen der Ventileinheit zu erzeugen. Vorzugsweise ist einer solchen Ausgestaltung die Ventileinheit als ein Drehschieberventil ausgebildet. Dann ist die Oszillatoreinrichtung insbesondere dazu geeignet und ausgebildet, die Schwingung durch ein wiederholtes Drehen wenigstens eines Ventilkolbens des Drehschieberventils in verschiedene Richtungen zu erzeugen. Dabei kann die Drehbewegung zwischen der ersten und zweiten und insbesondere auch der dritten Ventilstellung erfolgen. Möglich ist aber auch, dass die Drehbewegung nur innerhalb der ersten und/oder zweiten Ventilstellung erfolgt.

Es ist auch möglich, dass die Oszillatoreinrichtung die Schwingung mit einer anderen geeigneten Einrichtung erzeugt. Beispielsweise kann die Oszillatoreinrichtung dazu wenigstens eine drehbare Lochscheibe umfassen, welche in der Atemluftströmung angeordnet ist.

Die Oszillatoreinrichtung ist bevorzugt dazu geeignet und ausgebildet, während der Insufflation und/oder der Exsufflation die Ventileinheit zwischen einer vollständig geöffneten Ventilstellung und einer teilweise geöffneten Ventilstellung umzuschalten. Die Oszillatoreinrichtung ist bevorzugt dazu geeignet und ausgebildet, während der Insufflation und/oder der Exsufflation die Ventileinheit zwischen einer voll geöffneten Ventilstellung und einer voll geschlossenen Ventilstellung umzuschalten und/oder die Ventileinheit zwischen wenigstens zwei wenigstens teilweise geöffneten Ventilstellungen umzuschalten.

Die Oszillatoreinrichtung ist insbesondere dazu geeignet und ausgebildet, während der Insufflation und/oder der Exsufflation die Ventileinheit zwischen einer voll geöffneten und einer vollständig geschlossenen Ventilstellung umzuschalten. Die Oszillatoreinrichtung ist insbesondere dazu geeignet und ausgebildet, während der Insufflation und/oder der Exsufflation die Ventileinheit zwischen einer teilweise geöffneten und einer anderen teilweise geöffneten Ventilstellung oder einer vollständig geschlossenen Ventilstellung umzuschalten. Vorzugsweise ist die Oszillatoreinrichtung dazu geeignet und ausgebildet, während der Insufflation die Ventileinheit zwischen einer wenigstens teilweise geöffneten Ventilstellung für die Insufflation und einer wenigstens teilweise geöffneten Ventilstellung für die Exsufflation umzuschalten. Bevorzugt ist die Oszillatoreinrichtung dazu geeignet und ausgebildet, während der Exsufflation die Ventileinheit zwischen einer wenigstens teilweise geöffneten Ventilstellung für die Exsufflation und einer wenigstens teilweise geöffneten Ventilstellung für die Insufflation umzuschalten. Dadurch kann eine besonders zügige Druckeinstellung erfolgen und/oder eine Anpassung der Amplitude der Schwingung erfolgen. Beispielsweise kann ein besonders schneller und/oder ausgeprägter Druckabbau während der Insufflation erfolgen, wenn vorübergehend in die Ventilstellung für Exsufflation geschaltet wird.

Insbesondere ist die Oszillatoreinrichtung dazu geeignet und ausgebildet, wiederholt von dem ersten Lüfter auf den zweiten Lüfter zu schalten und umgekehrt. Besonders bevorzugt wird der erste Lüfter dabei mit einer vollständig geöffneten Ventilstellung zugeschaltet und der zweite Lüfter mit einer nur teilweise geöffneten Ventilstellung zugeschaltet und/oder umgekehrt. Insbesondere ist die Oszillatoreinrichtung dazu geeignet und ausgebildet, zwischen Atemluftströmungen mit jeweils einer anderen Strömungsrichtung umzuschalten. Vorzugsweise ist die Oszillatoreinrichtung dazu geeignet und ausgebildet, die Ventileinheit zwischen einer vollständig geöffneten ersten und/oder zweiten Ventilstellung und einer wenigstens teilweise geöffneten zweiten und/oder ersten Ventilstellung umzuschalten. Insbesondere erfolgt zur Oszillation ein Umschalten zwischen der ersten und der zweiten Ventilstellung. Dabei kann in der ersten und/oder zweiten Ventilstellung in eine Zwischenstellung geschaltet werden.

Das Umschalten im Rahmen der Oszillation kann also z. B. innerhalb der ersten und/oder zweiten Ventilstellung erfolgen oder z. B. von der ersten in die zweite Ventilstellung erfolgen. So kann eine besonders wirksame Schwingung erzeugt werden.

Es ist möglich und bevorzugt, dass die Oszillator Einrichtung dazu geeignet und ausgebildet ist, während der Insufflation einen anderen maximalen und/oder minimalen Öffnungsgrad der Ventileinheit als während der Exsufflation einzustellen. Dadurch können die Schwingungen besonders individuell für Insufflation und Exsufflation eingestellt werden. Beispielsweise erfolgt während der Insufflation ein Umschalten zwischen einer voll geöffneten und einer teilweise geöffneten Ventilstellung und während der Exsufflation ein Umschalten zwischen einer teilweise geöffneten zu einer anderen teilweise geöffneten Ventilstellung.

Die Oszillatoreinrichtung ist vorzugsweise dazu geeignet und ausgebildet, die Ventileinheit zur Erzeugung der Schwingung während der Insufflation und/oder Exsufflation mit einer Frequenz von 0,1 Hz bis 100 Hz umzuschalten. Besonders bevorzugt ist die Oszillatoreinrichtung dazu geeignet und ausgebildet, die Ventileinheit mit einer Frequenz von 1 Hz bis 30 Hz umzuschalten. Solche Frequenzen ermöglichen ein besonders effektives lösen des Sekrets. Möglich sind auch andere Frequenzbereiche. Besonders bevorzugt ist wenigstens ein Ventilkolben der Ventileinheit in dieser Frequenz bewegbar und insbesondere drehbar. Insbesondere ist das Drehschieberventil dazu geeignet und ausgebildet, einen Drehrichtungswechsel eines Ventilkolbens in einer solchen Frequenz auszuführen.

In einer bevorzugten Ausgestaltung ist die Oszillatoreinrichtung dazu geeignet und ausgebildet, für die Schwingung während der Insufflation eine andere Frequenz und/oder Amplitude als für die Schwingung während der Exsufflation einzustellen. Möglich ist auch, dass für die Insufflation und die Exsufflation die gleiche Frequenz und/oder Amplitude einstellbar ist.

Die Oszillation kann durch eine Benutzereingabe einstellbar sein. Beispielsweise kann die Frequenz und/oder Amplitude nach Benutzervorgaben angepasst werden. Die Einstellungen können vorzugsweise für die Insufflation und die Exsufflation separat vornehmbar sein.

In einer vorteilhaften Ausgestaltung umfasst die Atemluftschnittstelle wenigstens einen Lufteinlass und wenigstens einen Luftauslass. Vorzugsweise werden der Lufteinlass und der Luftauslass durch eine gemeinsame Öffnung bereitgestellt. Das ermöglicht eine besonders Bauraum sparende Unterbringung der Atemluftschnittstelle. Besonders vorteilhaft ist auch, dass vorhandene Gerätestrukturen mit einer einzelnen Öffnung weiterverwendet werden können. Beispielsweise können dadurch Beatmungsgeräte mit wenig Aufwand zu sehr hochwertigen und wirksamen Hustengeräten modifiziert werden.

Besonders vorteilhaft ist eine solche Ausgestaltung dann, wenn die Ventileinheit strömungstechnisch zwischen den wenigstens zwei Lüftern und der Atemluftschnittstelle angeordnet ist. Dann kann durch die Ventilstellung die Auswahl erfolgen, ob durch die gemeinsame Öffnung angesaugt oder ausgeblasen wird. Da somit kein zeitgleiches Ansaugen und Ausblasen vorgesehen ist, kann ein unbeabsichtigtes Ansaugen von ausgeblasener Luft sehr zuverlässig verhindert werden.

In einer ebenfalls bevorzugten Ausgestaltung umfasst die Patientenschnittstelle wenigstens eine Kupplungseinrichtung zum Anschließen wenigstens einer Schlaucheinrichtung. Die Schlaucheinrichtung ist mit wenigstens einer Atemöffnung des Patienten verbindbar. Die Schlaucheinrichtung umfasst wenigstens einen Einatemschlauch und wenigstens einen Ausatemschlauch. Die Schlaucheinrichtung kann auch nur wenigstens einen Einatemschlauch umfassen. Dann ist insbesondere kein Ausatemschlauch vorgesehen. Solche Schlaucheinrichtungen ermöglichen eine besonders gute Unterstützung bei der Sekretabfuhr. Das Atemtherapiegerät kann wenigstens eine Schlaucheinrichtung umfassen.

Beispielsweise kann die Schlaucheinrichtung als ein Leckageschlauchsystem ausgebildet sein, welches durch die Integration wenigstens einer Leckageöffnung gekennzeichnet ist. Die Schlaucheinrichtung kann auch mit wenigstens einem Ventil ausgestattet sein, welches beim Ausatmen bzw. Husten umschaltbar ist.

Insbesondere sind die wenigstens zwei Lüfter an der Patientenschnittstelle strömungsverbunden. Das ermöglicht eine gemeinsame Kupplungseinrichtung für beide Lüfter. Dann kann beispielsweise je nach Ventilstellung die Atemluftströmung für die Insufflation oder für die Exsufflation an der Kupplungseinrichtung bereitgestellt werden.

Möglich ist auch, dass für den ersten und den zweiten Lüfter jeweils wenigstens eine Kupplungseinrichtung vorgesehen ist. Dann sind beispielsweise der erste Lüfter mit dem Ausatemschlauch und der zweite Lüfter mit dem Einatemschlauch separat verbunden oder umgekehrt.

Die Strömungseinrichtung ist bevorzugt dazu geeignet und ausgebildet, mittels der Ventileinheit und/oder mittels wenigstens eines der wenigstens zwei Lüfter nachfolgend zu der Atemluftströmung für die Exsufflation wenigstens eine Atemluftströmung mit wenigstens einem definierten positiven Therapiedruck zur Unterstützung der Atmung für eine definierte Zeit einzustellen. Das ermöglicht eine für den Patienten sehr angenehme Erholungspause zwischen den Hustenvorgängen. Insbesondere ist die Strömungseinrichtung dazu geeignet und ausgebildet, nachfolgend zu der Atemluftströmung für die Exsufflation wenigstens eine Atemluftströmung für eine Beatmung einzustellen. Dadurch kann der Patient mit dem Hustengerät verbunden bleiben und weiter atmen. Die Strömungseinrichtung kann dazu geeignet und ausgebildet sein, eine Atemluftströmung mit wenigstens einem Druckprofil zu erzeugen.

Insbesondere ist die Strömungseinrichtung dazu geeignet und ausgebildet, für den definierten positiven Therapiedruck eine Atemluftströmung mit einem PEEP (Positive End-Expiratory Pressure) und/oder mit einem CPAP (Continous Positive Airway Pressure) einzustellen. Die Atemluftströmung mit dem PEEP bzw. CPAP während der Pause ist für die Atmung besonders hilfreich. Insbesondere wird die Atemluftströmung für den positiven Therapiedruck und vorzugsweise für den PEEP und/oder CPAP mit demjenigen Lüfter erzeugt, welcher zur Erzeugung der Atemluftströmung für die Insufflation vorgesehen ist. Dieser Lüfter ist insbesondere dann durch die Ventileinheit zugeschaltet, wenn die Atemluftströmung für den Staudruck erzeugt wird. Zur Erzeugung des positiven Therapiedrucks erfolgt vorzugsweise wenigstens eine Drehzahlanpassung wenigstens eines der wenigstens zwei Lüfter.

Insbesondere ist für den positiven Therapiedruck und vorzugsweise für den PEEP und/oder den CPAP ein geringerer Volumenfluss und/oder Druck als für die Insufflation vorgesehen. Beispielsweise beträgt der Volumenfluss und/oder Druck der Atemluftströmung für den positiven Therapiedruck weniger als die Hälfte des Volumenflusses und/oder des Drucks der Atemluftströmung für die Insufflation.

Es ist möglich, dass das Atemtherapiegerät wenigstens eine Beatmungseinrichtung umfasst. Die Beatmungseinrichtung ist insbesondere dazu geeignet und ausgebildet, mittels der Strömungseinrichtung eine Atemluftströmung zur Beatmung des Patienten zu erzeugen. Eine solche Ausgestaltung ist besonders vorteilhaft, da das Atemtherapiegerät zusätzlich zur Unterstützung der Sekretabfuhr auch für eine Beatmung des Patienten eingesetzt werden kann. Patienten benötigen oft eine Unterstützung bei der Sekretabfuhr in Kombination mit einer Beatmung. Durch die Integration einer Beatmungseinrichtung in das erfindungsgemäße Atemtherapiegerät können diese Aufgaben durch ein einzelnes Gerät bereitgestellt werden. Zudem kann das Atemtherapiegerät besonders unaufwendig in ein bestehendes Beatmungsgerät integriert werden. Es kann auch besonders unaufwendig ein bestehendes Beatmungsgerät in das Atemtherapiegerät integriert werden.

Die Beatmungseinrichtung umfasst insbesondere wenigstens eine Steuereinrichtung zur Ansteuerung der Strömungseinrichtung und/oder zur Einstellung von Beatmungsparametern. Die Beatmungseinrichtung und die Strömungseinrichtung können auch wenigstens eine gemeinsame Steuereinrichtung aufweisen. Die Steuereinrichtung umfasst beispielsweise wenigstens einen Prozessor und/oder Controller und/oder wenigstens einen Algorithmus bzw. eine Software.

Die Beatmungseinrichtung ist insbesondere dazu geeignet und ausgebildet, mittels der Strömungseinrichtung eine Atemluftströmung zur Inspiration und/oder zur Exspiration und/oder einen positiven Therapiedruck zu erzeugen. Insbesondere ist die Beatmungseinrichtung dazu geeignet und ausgebildet, mittels der Strömungseinrichtung die Atemluftströmung für die Insufflation als Atemluftströmung zur Inspiration bzw. zur Beatmung des Patienten einzusetzen. Ein Gerät mit einer solchen Beatmungseinrichtung ermöglicht eine Beatmung, welche besonders gut an die Bedürfnisse des Patienten angepasst werden kann.

In einer solchen Ausgestaltung ist die Patientenschnittstelle besonders bevorzugt mit wenigstens einer Schlaucheinrichtung zur Beatmung koppelbar. Beispielsweise kann eine Schlaucheinrichtung zur Leckagebeatmung und/oder Ventilbeatmung vorgesehen sein.

Vorzugsweise ist die Beatmungseinrichtung dazu geeignet und ausgebildet, im Rahmen einer Beatmung einen negativen Therapiedruck zu erzeugen, sodass ein Ausatemvorgang mit einem Unterdruck unterstützt werden kann. Dadurch kann die Ausatemluft schneller abgeführt werden bzw. die Ausatmung erheblich einfacher für den Patienten erfolgen. Ein besonderer Vorteil daran ist, dass dadurch Sekret gelöst bzw. abgeführt werden kann, ohne dass ein Husten nötig ist. Das reduziert die Belastung für den Patienten erheblich. So ist zum Beispiel eine Sekrethilfe im Rahmen einer an sich insgesamt normalen Beatmung möglich. Anhand der zwei parallel geschalteten Lüfter bzw. der daran gekoppelten schaltbaren Ventileinheit kann eine solche Ausgestaltung besonders vorteilhaft umgesetzt werden.

Der negative Therapiedruck entspricht insbesondere einem Unterdruck an der Patientenschnittstelle und/oder in der Schlaucheinrichtung und/oder in den Atemwegen des Patienten. Der negative Therapiedruck ist insbesondere durch wenigstens eine Erhöhung der Spitzenflüsse gekennzeichnet. Der negative Therapiedruck wird insbesondere mit dem Lüfter und/oder mit der Ventilstellung erzeugt, welche auch zur Erzeugung der Atemluftströmung für die Exsufflation vorgesehen ist. Möglich ist auch eine andere Erzeugung des negativen Therapiedrucks.

In der zuvor genannten Ausgestaltung ist die Patientenschnittstelle bevorzugt mit wenigstens einer Schlaucheinrichtung koppelbar, welche wenigstens einen Einatemschlauch und wenigstens einen Ausatemschlauch umfasst. Die Atemluftströmung für die Insufflation ist insbesondere durch den ersten Lüfter erzeugbar und/oder die Atemluftströmung für die Exsufflation ist insbesondere durch den zweiten Lüfter erzeugbar. Möglich ist auch, dass Atemluftströmung für die Insufflation durch den zweiten Lüfter erzeugbar ist und/oder dass die Atemluftströmung für die Exsufflation durch den ersten Lüfter erzeugbar ist.

Das Verfahren dient zum Betreiben eines Atemtherapiegeräts zur gezielten Unterstützung einer Sekretabfuhr aus den Atemwegen eines Patienten. Mit wenigstens einer Strömungseinrichtung werden wenigstens eine Atemluftströmung für eine Insufflation in den Patienten und wenigstens eine Atemluftströmung für eine Exsufflation aus dem Patienten erzeugt. Die Strömungseinrichtung umfasst wenigstens eine Patientenschnittstelle zum Anbinden des Patienten an das Atemtherapiegerät und wenigstens eine Atemluftschnittstelle zum Anbinden des Atemtherapiegeräts an Atemluft bzw. Umgebungsluft. Dabei umfasst die Strömungseinrichtung wenigstens zwei strömungstechnisch parallel geschaltete und mit wenigstens einer schaltbaren Ventileinheit strömungsverbundene Lüfter. Dabei wird die Ventileinheit in wenigstens eine erste Ventilstellung geschaltet. In der ersten Ventilstellung wird wenigstens ein erster Lüfter mit der Patientenschnittstelle und der Atemluftschnittstelle strömungsverbunden. Dadurch wird die Atemluftströmung für die Insufflation oder Exsufflation bereitgestellt.

Auch das Verfahren bietet eine besonders wirksame Unterstützung bei der Sekretabfuhr. Durch das Umschalten der Ventileinheit und dem Zuschalten des Lüfters kann ein besonders kurzfristiger Wechsel zwischen Insufflation und Exsufflation erfolgen. Dadurch wird der Hustenvorgang besonders gut unterstützt bzw. simuliert. Vorzugsweise wird in der ersten Ventilstellung wenigstens ein zweiter Lüfter von der Patientenschnittstelle und/oder der Atemluftschnittstelle strömungstechnisch wenigstens teilweise und vorzugsweise vollständig getrennt.

In einer vorteilhaften Ausgestaltung wird die Ventileinheit vorzugsweise in wenigstens eine zweite Ventilstellung geschaltet. In der zweiten Ventilstellung wird insbesondere wenigstens ein zweiter Lüfter mit der Patientenschnittstelle und der Atemluftschnittstelle strömungsverbunden. Dadurch wird vorzugsweise die Atemluftströmung für die Exsufflation bereitgestellt, wenn in der ersten Ventilstellung die Atemluftströmung für die Insufflation bereitgestellt wird. Möglich ist auch, dass dadurch die Atemluftströmung für die Insufflation bereitgestellt wird, wenn in der ersten Ventilstellung die Atemluftströmung für die Exsufflation bereitgestellt wird.

In der zweiten Ventilstellung wird der erste Lüfter von der Patientenschnittstelle und/oder der Atemluftschnittstelle strömungstechnisch wenigstens teilweise und vorzugsweise vollständig getrennt.

Bevorzugt wird die Ventileinheit in wenigstens eine dritte Ventilstellung geschaltet. Insbesondere werden der erste Lüfter und der zweite Lüfter in der dritten Ventilstellung von der Patientenschnittstelle und/oder der Atemluftschnittstelle strömungstechnisch wenigstens teilweise und vorzugsweise vollständig getrennt.

Vorzugsweise können in der ersten und/oder zweiten und/oder dritten Ventilstellung Zwischenstellungen geschaltet werden. Vorzugsweise wird das zuvor beschriebene Atemtherapiegerät nach dem Verfahren betrieben. Insbesondere ist das zuvor beschriebene Atemtherapiegerät dazu geeignet und ausgebildet, nach dem Verfahren betrieben zu werden.

Die Anmelderin behält sich vor, ein Atemtherapiegerät und insbesondere ein Hustengerät zu beanspruchen, welches zur gezielten Unterstützung einer Sekretabfuhr aus den Atemwegen eines Patienten dient und wenigstens eine Strömungseinrichtung umfasst. Die Strömungseinrichtung dient insbesondere zum Erzeugen wenigstens einer Atemluftströmung für eine Insufflation in den Patienten und insbesondere zum Erzeugen wenigstens einer Atemluftströmung für eine Exsufflation aus dem Patienten. Die Strömungseinrichtung umfasst insbesondere wenigstens eine Patientenschnittstelle zum Anbinden des Patienten an das Atemtherapiegerät. Die Strömungseinrichtung umfasst insbesondere wenigstens eine Atemluftschnittstelle zum Anbinden des Atemtherapiegeräts an Atemluft bzw. Umgebungsluft. Dabei ist die Strömungseinrichtung vorzugsweise dazu geeignet und ausgebildet, mittels wenigstens eines Lüfters und/oder wenigstens einer Ventileinheit nachfolgend zu der Atemluftströmung für die Exsufflation wenigstens eine Atemluftströmung für eine Beatmung mit wenigstens einem definierten positiven Therapiedruck zur Unterstützung des Ausatemvorgangs für eine definierte Zeit einzustellen.

Ein solches Atemtherapiegerät bietet eine für den Patienten sehr angenehme Erholungspause zwischen den Hustenvorgängen. Der Lüfter kann dabei der zuvor beschriebene erste oder zweite Lüfter sein. Der Lüfter und/oder die Ventileinheit sind vorzugsweise wie zuvor beschrieben ausgebildet.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In der Figur zeigt:
- Fig. 1: eine stark schematische Darstellung eines erfindungsgemäßen Atemtherapiegeräts;
- Fig. 2: eine stark schematische Darstellung eines weiteren Atemtherapiegeräts;
- Fig. 3: eine stark schematische Darstellung eines anderen Atemtherapiegeräts;
- Fig. 4: eine schematische Darstellung einer Ventileinrichtung in einer perspektivischen Ansicht;
- Fig. 5-9: schematische Darstellungen der Ventileinrichtung in verschiedenen Stellungen in einer geschnittenen Ansicht;
- Fig. 10: ein stark schematisches Schaubild zur Funktionsweise des Atemtherapiegeräts; und
- Fig. 11: ein weiteres stark schematisches Schaubild zur Funktionsweise des Atemtherapiegeräts.

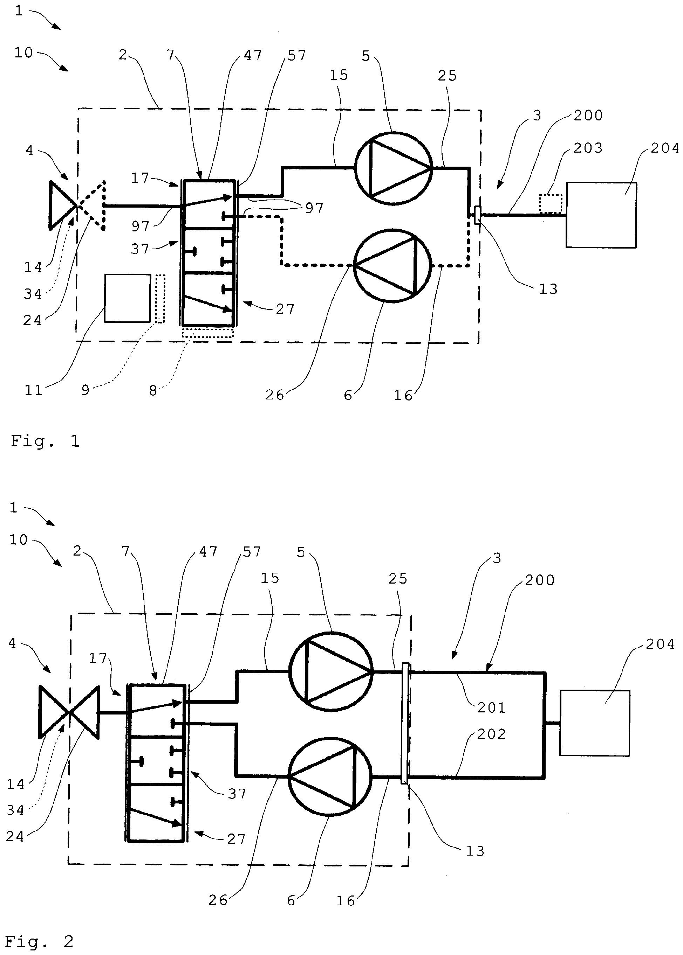

Die Figur 1 zeigt ein erfindungsgemäßes Atemtherapiegerät 1, welches als Hustengerät 10 ausgebildet ist. Das Atemtherapiegerät 1 wird hier nach dem Verfahren betrieben und dient zur gezielten Unterstützung einer Sekretabfuhr aus den Atemwegen eines Patienten.

Das Atemtherapiegerät 1 umfasst eine Strömungseinrichtung 2, mit welcher eine Atemluftströmung für eine Insufflation und eine Atemluftströmung für eine Exsufflation erzeugt wird. Die Strömungseinrichtung 2 umfasst einen ersten Lüfter 5, einen zweiten Lüfter 6, eine Ventileinheit 7 und eine Patientenschnittstelle 3 sowie eine Atemluftschnittstelle 4. Die Lüfter 5, 6 sind parallel geschaltet und können separat angesteuert werden. Zur Ansteuerung der Ventileinheit 7 und der Lüfter 5, 6 umfasst das Atemtherapiegerät 1 hier eine Steuereinrichtung 11.

Die Ventileinheit 7 umfasst hier drei Anschlüsse 97 und bietet drei schaltbare Ventilstellungen 17, 27, 37. Die Ventileinheit 7 ist als ein 3/3-Wegeventil 47 ausgebildet. Zudem ist die Ventileinheit 7 hier als Proportionalventil 57 ausgebildet, sodass in den Ventilstellungen 17, 27 Zwischenstellungen mit verschiedenen Öffnungsgraden eingestellt werden können.

Der erste Lüfter 5 ist mit einer Ansaugseite 15 mit einem Anschluss 97 der Ventileinheit 7 verbunden. Die Abgabeseite 25 bzw. Druckseite des ersten Lüfters 5 ist hier mit der Patientenschnittstelle 3 verbunden. Der zweite Lüfter 6 ist mit seiner Abgabeseite 26 mit einem zweiten Anschluss 97 der Ventileinheit 7 verbunden. Die Ansaugseite 16 des zweiten Lüfters 6 ist hier mit der Patientenschnittstelle 3 verbunden. Die Ventileinheit 7 umfasst einen dritten Anschluss 97, welcher hier mit der Atemluftschnittstelle 4 verbunden ist.

Die Atemluftschnittstelle 4 ist mit der Umgebung des Atemtherapiegeräts 1 strömungsverbunden, sodass Umgebungsluft angesaugt und für die Insufflation eingesetzt werden kann. Dazu weist die Atemluftschnittstelle 4 einen Lufteinlass 14 auf. Alternativ oder zusätzlich kann die Atemluftschnittstelle 4 auch mit einer Atemgasquelle und beispielsweise einer Druckflasche verbunden sein.

Die Atemluftschnittstelle 4 ist zudem mit einem Luftauslass 24 ausgestattet, über welchen die während der Exsufflation abgesaugte Luft in die Umgebung des Geräts 1 ausgeblasen werden kann.

Bevorzugt werden Lufteinlass 14 und Luftauslass 24 durch eine gemeinsame Öffnung 34 bereitgestellt, über welche die Luft sowohl eingesaugt als auch ausgeblasen werden kann. Es können auch separate Öffnungen für Lufteinlass 14 und Luftauslass 24 vorgesehen sein.

Die Patientenschnittstelle 3 ist hier mit einer Kupplungseinrichtung 13 ausgestattet, an welche eine Schlaucheinrichtung 200 koppelbar ist. Die Schlaucheinrichtung 200 ist mit einem Patienteninterface 204 ausgestattet. Das Patienteninterface 204 kann beispielsweise als eine Vollgesichtsmaske, eine Nasalmaske, ein Nasal-Pillow, ein Mundstück, ein Tubus oder als eine Larynxmaske ausgestaltet sein. Zur Fixierung der Atemmaske 105 kann eine Kopfhaube vorgesehen sein.

Die hier gezeigte Schlaucheinrichtung 200 ist nur mit einem Atemschlauch ausgestattet, über welchen die Atemluft sowohl für die Insufflation als auch für die Exsufflation gefördert wird. Beispielsweise ist ein Einschlauchsystem vorgesehen, welches für Hustenmanöver geeignet ist. Dann ist beispielsweise eine Rückatmung in die Schlaucheinrichtung 200 möglich. Es ist möglich, dass auf eine CO2-Auswaschung im Bereich des Schlauchsystems verzichtet wird. Es ist aber auch ein Zweischlauchsystem koppelbar.

In einer Ausgestaltung kann die Schlaucheinrichtung 200 mit einem Patientenventil 203 ausgestattet sein. Darüber kann beispielsweise kontinuierlich Ausatemluft abführbar sein. Das Patientenventil 203 kann beispielsweise als passives Ausatemsystem zur CO2-Auswaschung ausgestaltet sein.

Es kann aber auch ein durch das Atemtherapiegerät steuerbares Patientenventil 203 vorgesehen sein, sodass das Abführen von Ausatemluft gezielt an Atemphasen bzw. Hustenphasen angepasst werden kann.

Erfindungsgemäß umfasst das Atemtherapiegerät 1 auch eine hier nicht näher dargestellte Sensoreinrichtung, welche den Volumendurchfluss bzw. Flow und/oder den Druck der Atemgasströmung für die Insufflation bzw. Exsufflation überwacht. Dazu kann die Sensoreinrichtung entsprechende Drucksensoren und/oder Flowsensoren aufweisen. Die Steuereinrichtung 11 ist erfindungsgemäß dazu geeignet und ausgebildet, in Abhängigkeit der Sensorsignale die Ventileinheit 7 und/oder die Lüfter 5, 6 in Abhängigkeit der erfassten Sensorsignale einzustellen bzw. zu regeln. Auch das Patientenventil 203 kann durch die Steuereinrichtung 11 ansteuerbar sein.

Die hier gezeigten Strömungswege können mit wenigstens einer Filtereinrichtung ausgestattet sein, um eine aufgereinigte Atemluftströmung zur Verfügung stellen zu können.

In einer vorteilhaften Ausgestaltung kann das Atemtherapiegerät 1 auch mit einer Beatmungseinrichtung 9 ausgestattet sein. Die Beatmungseinrichtung 9 ist dann mit der Strömungseinrichtung 2 wirkverbunden, um damit eine Atemluftströmung zur Beatmung des Patienten zu erzeugen. Dazu kann die Beatmungseinrichtung 9 wenigstens einen der Lüfter 5, 6 und/oder die Ventileinheit 7 entsprechend ansteuern. Vorzugsweise ist die Beatmungseinrichtung 9 auch mit der Steuereinrichtung 11 und der Sensoreinrichtung wirkverbunden.

Ein einer Ausgestaltung der Beatmungseinrichtung 9 ist in der Steuereinrichtung 11 dazu wenigstens eine Software hinterlegt, anhand derer die Strömungseinrichtung 2 angesteuert wird. Das bietet einen besonders vorteilhaften Konstruktionsaufwand. Die Beatmungseinrichtung 9 kann auch eine eigene bzw. separate Steuereinrichtung aufweisen.

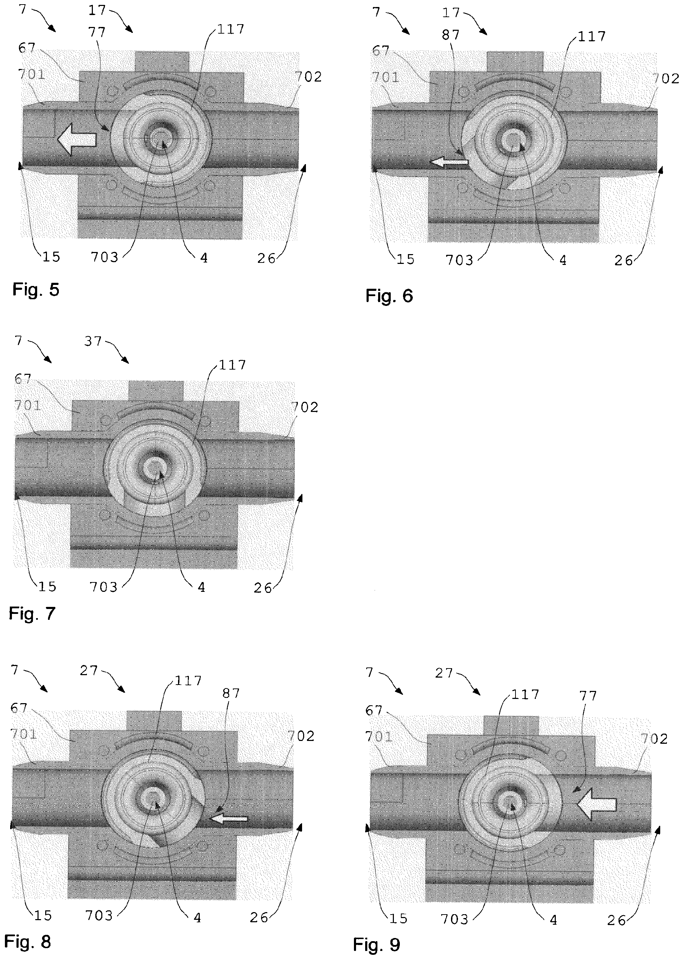

Das Atemtherapiegerät 1 kann auch mit einer Oszillatoreinrichtung ausgestattet sein, um die Atemluftströmung für die Insufflation und Exsufflation mit einer definierten Schwingung zu beaufschlagen. Beispielsweise wird die Oszillatoreinrichtung 8 durch die Ventileinheit 7 zur Verfügung gestellt. Eine solche Ausgestaltung ist mit Bezug zu den Figuren 5 bis 10 näher beschrieben.

Das Atemtherapiegerät 1 kann weitere, hier nicht näher dargestellte Komponenten aufweisen. Beispielsweise können eine Anzeigeeinrichtung bzw. ein Display sowie eine Bedieneinheit zur Vornahme von Eingaben und Einstellungen vorgesehen sein. Zudem kann das Atemtherapiegerät 1 wenigstens eine Kommunikationsschnittstelle aufweisen, über welche es mit externen Geräten drahtlos und/oder drahtgebunden kommunizieren kann. Zudem kann auch eine Fernsteuerung für das Atemtherapiegerät 1 vorgesehen sein. Solche Komponenten sind vorzugsweise mit der Steuereinrichtung 11 wirkverbunden.

In der Steuereinrichtung 11 sind vorzugsweise Vorgaben zur Ansteuerung der Ventileinheit 7 bzw. der Lüfter 5, 6 hinterlegt bzw. gespeichert. Diese Vorgaben können insbesondere durch den Benutzer bzw. einen Betreuer wenigstens teilweise angepasst werden. Die Steuereinrichtung 11 umfasst beispielsweise wenigstens einen Controller und/oder andere Steuerkomponenten. Die Ventileinheit 7 ist hier in der ersten Ventilstellung 17 gezeigt. Dabei ist der erste Lüfter 5 an seiner Ansaugseite 15 mit der Atemluftschnittstelle 4 verbunden. So kann der erste Lüfter 5 Luft aus der Umgebung des Geräts 1 ansaugen und als Atemluftströmung für die Insufflation an der Patientenschnittstelle 3 bereitstellen. Von dort kann die Atemluftströmung über die Schlaucheinrichtung 200 und das Patienteninterface 204 dann zur Insufflation in den Patienten eingeblasen werden.

Der zweite Lüfter 6 kann in der ersten Ventilstellung 17 aktiviert oder auch deaktiviert sein. Da der zweite Lüfter 6 in der ersten Ventilstellung 17 gegenüber der Atemluftschnittstelle 4 gesperrt ist, kommt es auch bei einem Betrieb des Lüfters 6 zu keiner unerwünschten Exsufflation während der vorgesehenen Insufflation. So kann der zweite Lüfter 6 bereits während der Insufflation auf eine für die anstehende Exsufflation besonders günstige Drehzahl hochgefahren werden.

Dieser Vorteil liegt in der zweiten Ventilstellung 27 ebenfalls vor. Dann kann der erste Lüfter 5 auf eine gewünschte Drehzahl gebracht werden, ohne die mit dem zweiten Lüfter 6 durchgeführte Exsufflation zu beeinträchtigen.

In der zweiten Ventilstellung 27 ist hier die Abgabeseite 26 des zweiten Lüfters 6 mit der Atemluftschnittstelle 4 verbunden. Der zweite Lüfter 6 kann über die Patientenschnittstelle 3 und die daran gekoppelte Schlaucheinrichtung 200 sowie das Patienteninterface 204 während der Exsufflation Luft aus dem Patienten absaugen. Der zweite Lüfter 6 bläst die abgesagte Luft dann über die Atemluftschnittstelle 4 in die Umgebung des Geräts 1 ab.

In der dritten Ventilstellung 37 ist hier keiner der Lüfter 5, 6 mit der Atemluftschnittstelle 4 verbunden bzw. beide Lüfter 5, 6 sind abgesperrt.

In der Figur 2 ist das Atemtherapiegerät 1 mit einer alternativ ausgestalteten Patientenschnittstelle 3 gezeigt. Die Patientenschnittstelle 3 ist hier mit einer Kupplungseinrichtung 13 ausgestattet, an welcher eine Schlaucheinrichtung 200 mit zwei Schläuchen anschließbar ist. Dazu ist die Schlaucheinrichtung 200 hier mit einem Einatemschlauch 201 und einem Ausatemschlauch 202 ausgestattet, welche an das Patienteninterface 204 gekoppelt sind. Der Einatemschlauch 201 ist hier mit dem ersten Lüfter 5 gekoppelt, sodass darüber die Atemluftströmung für die Insufflation strömen kann. Der Ausatemschlauch 202 ist hier mit dem zweiten Lüfter 6 gekoppelt, sodass darüber die Atemluftströmung für die Exsufflation strömen kann. Beispielsweise umfasst das Patienteninterface 104 ein patientennahes Y-Stück. Dort können die beide Schläuche 201, 202 verbunden sein.

Ein solches Zweischlauchsystem kann auch besonders gut zur Beatmung eingesetzt werden. Das ist dann besonders vorteilhaft, wenn das Atemtherapiegerät 1 auch mit einer Beatmungseinrichtung 9 ausgestattet ist. Dies ist ebenfalls besonders vorteilhaft, wenn die CO2-reiche Ausatemluft nicht wieder eingeatmet werden soll.

Eine solche Ausführung des Atemtherapiegerätes 1 mit einem Zweischlauchsystem ist in dieser Hinsicht besonders vorteilhaft: Zur Leitung der Atemluft während einer Insufflation/Einatmung hin zum Patienten durch die Schlauchverbindung 201 und während einer Exsufflation/Ausatmung weg vom Patienten durch die Schlauchverbindung 202 wird nahezu keine CO2-reiche Atemluft wieder eingeatmet. Die integrierte Ventileinheit alleine steuert dabei die Flowrichtung und Durchströmung der Schlauchverbindungen in Abhängigkeit von den Atemphasen, ohne dass ein zusätzliches Patientenventil verwendet werden muss. Das ist ein besonderer Vorteil der Erfindung, welcher sich insbesondere durch die zwei parallel geschalteten Lüfter bzw. der daran gekoppelten schaltbaren Ventileinheit ergibt.

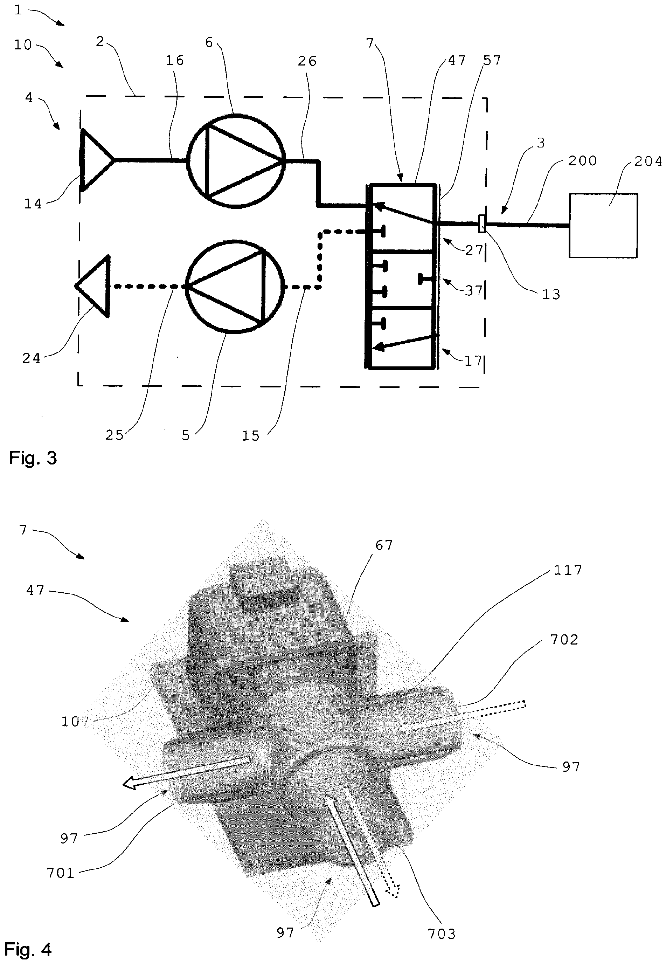

Die Figur 3 zeigt eine alternative Ausgestaltung des Atemtherapiegeräts 1. Die Ventileinheit 7 ist hier zwischen den Lüftern 5, 6 und der Patientenschnittstelle 3 angeordnet. Dabei ist die Ansaugseite 15 des ersten Lüfters 5 mit der Ventileinheit 7 verbunden. Die Abgabeseite 25 des ersten Lüfters 5 ist hier mit einem Luftauslass 24 der Atemluftschnittstelle 4 verbunden. So kann mittels der Ventileinheit 7 entweder der erste Lüfter 5 oder der zweite Lüfter 6 mit der Patientenschnittstelle 3 verbunden werden. Der zweite Lüfter 6 ist mit seiner Abgabeseite 26 an die Ventileinheit 7 angebunden. Die Ansaugseite 16 des zweiten Lüfters 6 ist hier mit einem Lufteinlass 14 der Atemluftschnittstelle 4 verbunden. Während zwei der Anschlüsse 97 also mit den Lüftern 5, 6 gekoppelt sind, ist der dritte Anschluss 97 hier mit der Patientenschnittstelle 3 verbunden. Die Ventileinheit 7 befindet sich hier in der zweiten Ventilstellung 27. Dabei ist der zweite Lüfter 6 mit der Patientenschnittstelle 3 verbunden. So kann der Lüfter 6 die Luft über den Lufteinlass 14 ansaugen und über die Patientenschnittstelle 3 und die daran angeschlossene Schlaucheinrichtung bzw. das Patienteninterface 204 zur Insufflation in den Patienten einblasen. Der erste Lüfter 5 ist in dieser Ventilstellung 27 gegenüber der Patientenschnittstelle 3 abgesperrt.

In der zweiten Ventilstellung 27 kann der erste Lüfter 5 weiterbetrieben werden, ohne dass eine unerwünschte Exsufflation zustande kommen würde. So kann der erste Lüfter 5 beispielsweise während der Insufflation bereits auf eine optimale Drehzahl hochgefahren werden, die für die nächste Exsufflation benötigt wird.

In der ersten Ventilstellung 17 ist der erste Lüfter 5 hier mit der Patientenschnittstelle 3 verbunden. Der zweite Lüfter 6 ist dann von der Patientenschnittstelle 3 abgesperrt. Der erste Lüfter 5 erzeugt dann eine Atemluftströmung für die Exsufflation und saugt dazu Luft über die Patientenschnittstelle 3 bzw. die Schlaucheinrichtung 200 und das Patienteninterface 204 aus dem Patienten. Der erste Lüfter 5 bläst die Luft über den Luftauslass 24 aus dem Gerät 1 in die Umgebung ab. Die erste Ventilstellung 17 ermöglicht eine Anpassung der Drehzahl des zweiten Lüfters 6, ohne die Exsufflation ungünstig zu beeinträchtigen.

In der Figur 4 ist eine Ausgestaltung der Ventileinheit 7 als ein Drehschieberventil 67 gezeigt. Das Drehschieberventil 67 ist hier als ein 3/3-Wegeventil 47 und kann als ein Proportionalventil 57 eingesetzt werden.

Das Drehschieberventil 67 umfasst hier drei Anschlüsse 97 und kann in drei Ventilstellungen gebracht werden. Die Ventilstellungen entsprechen beispielsweise den in der Figur 1 oder der Figur 3 gezeigten Ventilstellungen 17, 27, 37. So kann mit der Ventileinheit 7 entweder der erste Lüfter 5 oder der zweite Lüfter 6 mit der Atemluftschnittstelle 4 bzw. der Patientenschnittstelle 3 verbunden werden.

Zum Schalten der Ventilstellungen ist hier ein drehbarer Ventilkolben 117 vorgesehen, welcher mittels einer Antriebseinheit 107 in die jeweilige Position verfahren wird. Zur besseren Veranschaulichung des Drehkolbens 117 ist die Ventileinheit 7 hier teilweise transparent dargestellt.

Das hier gezeigte Drehschieberventil 67 kann beispielsweise in der mit Bezug zur Figur 1 beschriebenen Strömungseinrichtung 2 eingesetzt werden.

Dann ist an einem ersten Anschluss 701 die Ansaugseite 15 des ersten Lüfters 5 angeschlossen. An einem zweiten Anschluss 702 ist die Abgabeseite 26 des zweiten Lüfters 6 angeschlossen. An einem dritten Anschluss 703 ist die Atemschnittstelle 4 mit dem Lufteinlass 14 bzw. Luftauslass 24 angebunden. So kann durch Drehen des Ventilkolbens 117 entweder der erste Lüfter 5 oder der zweite Lüfter 6 mit der Atemschnittstelle 4 verbunden werden.

In der hier gezeigten Stellung des Ventilkolbens 117 ist der erste Lüfter 5 zugeschaltet, sodass eine Insufflation erfolgen kann. Wird der Ventilkolben 117 entsprechend gedreht, kann der zweite Lüfter 6 die Luft über die Atemschnittstelle 4 ausgeblasen, sodass eine Exsufflation möglich ist. Die Atemluftströmung für die Insufflation ist hier durch zwei Pfeile mit durchgezogenen Linien angedeutet. Die Atemluftströmung für die Exsufflation ist hier durch gestrichelte Pfeile angedeutet.

Wird das hier gezeigte Drehschieberventil 67 in der Strömungseinrichtung 2 gemäß der Figur 3 eingesetzt, ändert sich die Belegung der Anschlüsse 97 entsprechend. Dann ist an dem ersten Anschluss 701 die Abgabeseite 25 des ersten Lüfters 5 angeschlossen. In der hier gezeigten Stellung des Ventilkolbens 117 ist dann eine Exsufflation vorgesehen. An dem zweiten Anschluss 702 ist dann die Ansaugseite 16 des zweiten Lüfters 6 angeschlossen. An dem dritten Anschluss 703 ist die Patientenschnittstelle 3 angebunden.

Im Folgenden wird die Arbeitsweise der in der Figur 4 gezeigten Ventileinheit 7 beispielhaft erläutert. Dazu sind in den Figuren 5 bis 9 verschiedene Ventilstellungen gezeigt. Die Ventileinheit 7 ist in einer geschnittenen Vorderansicht mit Blick auf den Ventilkolben 117 gezeigt.

Das hier gezeigte Drehschieberventil 67 kann durch Verdrehen des Ventilkolbens 117 in der ersten und zweiten Ventilstellung 17, 27 verschiedene Zwischenstellungen 77, 87 mit unterschiedlichen Öffnungsgraden einnehmen. Dabei sind beliebige bzw. diskrete Zwischenstellungen möglich. Es können auch stetige bzw. fest vorgegebene Zwischenstellungen vorgesehen sein. Die Ventileinheit 7 kann also für die Insufflation und Exsufflation Zwischenstellungen einnehmen, in denen durch eine Querschnittsreduktion des Strömungsweges der Flow entsprechend reduziert wird.

Die Figuren 5 und 6 zeigen die Ventileinheit 7 in der ersten Ventilstellung 17. Dabei ist die Ventileinheit 7 der Figur 5 in einer vollständig geöffneten Ventilstellung 77 und in der Figur 6 in einer teilweise geschlossenen Ventilstellung 87 gezeigt. Die Figur 7 zeigt die Ventileinheit 7 in der dritten Ventilstellung 37. Die Anschlüsse 97 sind hier versperrt.

In den Figuren 8 und 9 ist die Ventileinheit 7 in der zweiten Ventilstellung 27 gezeigt. Dabei zeigt die Figur 8 eine teilweise geöffnete Ventilstellung 87 und die Figur 9 eine vollständig geöffnete Ventilstellung 77.

Wenn die hier gezeigte Ventileinheit 7 in der mit Bezug zur Figur 1 beschriebenen Strömungseinrichtung 2 eingesetzt wird, ist die Ansaugseite 15 des ersten Lüfters 5 an dem ersten Anschluss 701 angebunden. An dem zweiten Anschluss 702 ist die Abgabeseite 96 des zweiten Lüfters angebunden. Die Atemluftschnittstelle 4 ist mit dem dritten Anschluss 703 verbunden.

Dann kann mit der vollständig geöffneten Ventilstellung 77 der Figur 5 ein maximaler Flow für die Insufflation erreicht werden. Der Strömungsweg zwischen dem ersten Lüfter 5 und der Atemschnittstelle 4 ist hier maximal freigegeben.

Die nur teilweise geöffnete Ventilstellung 87 der Figur 6 ermöglicht einen entsprechend reduzierten Flow für die Insufflation. Der Strömungsweg zwischen dem ersten Lüfter 5 und der Atemluftschnittstelle 4 ist hier gezielt eingeschränkt.

Bei der in der Figur 7 gezeigten dritten Ventilstellung 37 erfolgt weder eine Insufflation noch eine Exsufflation. Beide Lüfter 5, 6 sind von der Atemluftschnittstelle 4 getrennt.

Die in der Figur 8 gezeigte teilweise geöffnete Ventilstellung 87 in der zweiten Ventilstellung 27 ermöglicht eine eingeschränkte bzw. reduzierte Exsufflation. Der Strömungsweg zwischen zweitem Lüfter 6 und der Atemluftschnittstelle 4 ist hier nur teilweise freigegeben.

In der Figur 9 ist die Verbindung zwischen zweitem Lüfter 6 und Atemluftschnittstelle 4 maximal freigegeben, sodass eine maximale Atemluftströmung für die Exsufflation eingestellt werden kann.

Bei einer Integration des Drehschieberventils 67 in die in der Figur 3 beschriebene Strömungseinrichtung 2 ist die Anbindung der Anschlüsse 701, 702, 703 gemäß der in der Figur 3 gezeigten Verschaltung entsprechend geändert.

Das hier vorgestellte Drehschieberventil 67 kann auch zur gezielten Erzeugung einer Schwingung von Druck und/oder Fluss der Atemluftströmung eingesetzt werden. Dazu wird der Ventilkolben 117 mit einer vorgegebenen bzw. einstellbaren Frequenz zwischen verschiedenen Stellungen verschwenkt.

Für die Erzeugung einer Oszillation während der Insufflation können beispielsweise die in den Figuren 5 bis 8 gezeigten Ventilstellungen eingenommen werden.

Beispielsweise ist zunächst die in der Figur 5 gezeigte vollständig geöffnete Ventilstellung 77 vorgesehen. So liegt während der Insufflation zunächst ein maximal möglicher positiver Druck bzw. maximal möglicher Flow vor. Anschließend wird der Ventilkolben 117 in die in der Figur 6 gezeigten teilweise geöffneten Ventilstellung 87 verschwenkt. Dadurch kommt es zu einer gezielten Verminderung des Drucks bzw. Flows während der Insufflation.

Anschließend wird der Ventilkolben 117 in die in der Figur 7 gezeigte geschlossene Ventilstellung 37 Verfahren. Dadurch ergibt sich eine Atemluftströmung während der Insufflation mit einem Flow von im Wesentlichen null. Der an dem Patienteninterface 204 anliegende Druck kann dabei beispielsweise auf ein Niveau sinken, dass im Wesentlichen durch den Grad der Lungenfüllung bestimmt wird.

Anschließend wird der Ventilkolben 117 in die in der Figur 8 gezeigte teilweise geöffnete Ventilstellung 87 der zweiten Ventilstellung 27 verschwenkt. Dadurch wird die Abgabeseite 26 des für die Exsufflation vorgesehenen zweiten Lüfters 6 zugeschaltet. Dadurch kommt es in dieser Stellung zu einer gewünschten kurzzeitigen Absenkung des Drucks mit einer Flowumkehr während der Oszillation.

Nun folgt vorzugsweise eine Richtungsumkehr der Drehbewegung des Ventilkolbens 117. Dabei wird der Ventilkolben 117 in umgekehrter Reihenfolge in die zuvor beschriebenen Stellungen zurückgedreht, bis die in der Figur 5 gezeigte vollständig geöffnete Ventilstellung 77 der ersten Ventilstellung 17 erreicht ist.

Dann erfolgt die zuvor beschriebene Ventilbewegung erneut. Die Wiederholungsrate kann dabei beispielsweise zwischen 1 Hz und 30 Hz liegen.

Die Erzeugung einer Oszillation während der Exsufflation kann beispielsweise durch die in den Figuren 6 bis 9 gezeigten Ventilstellungen erreicht werden.

Beispielsweise ist der Ventilkolben 117 zunächst in der in Figur 9 gezeigten vollständig geöffneten Ventilstellung 77 der zweiten Ventilstellung 27 angeordnet. Das ermöglicht eine Exsufflation mit einem maximal möglichen negativen Druck bzw. einem maximalen Flow für die Exsufflation.

Anschließend wird der Ventilkörper 117 in die nur teilweise geöffnete Ventilstellung 87 der Figur 8 verschwenkt. Dadurch wird der Flow während der Exsufflation gezielt verringert. Dann wird der Ventilkolben 117 in die dritte Ventilstellung 37 der Figur 7 verfahren. Die Strömungswege für Exsufflation und Insufflation sind dann vollständig geschlossen. Der Flow beträgt im Wesentlichen null. Der an dem Patienteninterface 204 anliegende Druck kann beispielsweise auf ein Niveau ansteigen, dass im Wesentlichen durch den Grad der Lungenfüllung bestimmt wird.

Anschließend wird der ventilkolben 117 in die teilweise geöffnete Ventilstellung 87 der ersten Ventilstellung 17 verschwenkt, wie sie in Figur 6 gezeigt ist. Dadurch wird während der Exsufflation die Ansaugseite 15 des ersten Lüfters 5 gezielt zugeschaltet. Dadurch kommt es zu einer gewünschten kurzzeitigen Anhebung des Drucks mit einer entsprechenden Flowumkehr während der Oszillation.

Anschließend wird der Ventilkolben 117 wieder über die zuvor beschriebenen Stellungen in umgekehrter Reihenfolge zurückschwenkt.

Erreicht der Ventilkolben 117 die in der Figur 9 gezeigte Stellung, beginnt die Drehbewegung während der Exsufflation von Neuem. Vorzugsweise erfolgt die Umkehr der Drehbewegung ebenfalls mit einer Frequenz von 1 Hz bis 30 Hz.

Die Drehbewegung während der Oszillation erfolgt vorzugsweise zwischen einer Endlage voll offen und wenigstens teilweise geschlossen. Die Drehbewegung während der Oszillation kann aber auch zwischen beiden Endlagen jeweils voll offen erfolgen. Die Drehbewegung während der Oszillation erfolgt bevorzugt zwischen den Endlagen voll offen für eine Strömungsrichtung und teilgeöffnet in die andere Strömungsrichtung. So kann beispielsweise während der Insufflation ein größerer Abbau des Drucks gewährleistet werden. Die Angaben der Drehrichtung beziehen sich dabei insbesondere auf eine Strömungsrichtung. Zudem wird unter einer Endlage insbesondere eine wirksame Endlage verstanden. Die wirksame Endlage kann einem Anschlag entsprechen. Die wirksame Endlage kann unabhängig von einem Endanschlag sein, z. B. bei einem Ventil ohne Anschlag bzw. einem 360°-Ventil.

Es können auch andere als die hier gezeigten Ventilstellungen für die Oszillation während der Insufflation und/oder Exsufflation vorgesehen sein.

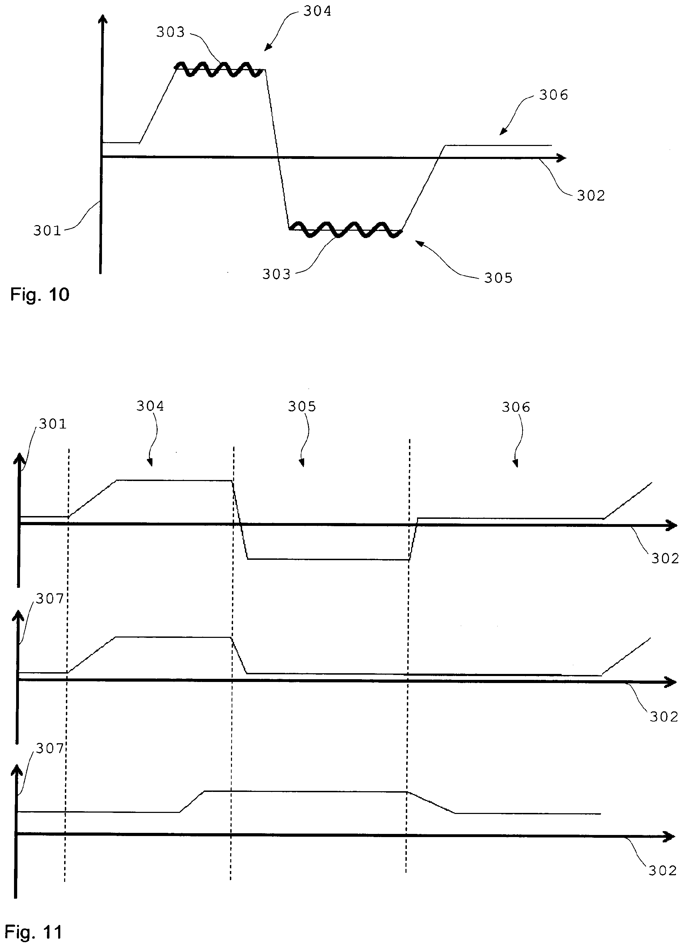

Die Figur 10 zeigt ein Beispiel eines Druckverlaufs, wie er bei einem Einsatz des Atemtherapiegeräts 1 vorgesehen sein kann. Dazu wurde der Druck 301 gegen die Zeit 302 aufgetragen.

Während der Insufflation 304 liegt für eine definierte Zeit ein entsprechend hoher Druck 301 vor. Für eine besonders wirksame Anregung des Hustenreizes bzw. zum besonders wirksamen Lösen des Sekrets wird dann sehr kurzfristig auf die Exsufflation 305 umgeschaltet. Dazu wird der Druck 301 innerhalb einer definierten Zeitspanne auf ein entsprechend negatives Niveau abgesenkt und für eine bestimmte Dauer gehalten.

Dann kann beispielsweise eine erneute Anhebung des Drucks auf das gewünschte Niveau für die Insufflation erfolgen. Anschließend erfolgt für die Exsufflation wieder eine sehr zügige Absenkung des Drucks 301. Dieses Wechseln zwischen Insufflation und Exsufflation kann für einen gewünschten Zeitraum wiederholt werden. Beispielsweise kann die Anzahl der Wiederholungen und/oder die Frequenz der Wiederholungen durch einen Benutzer bzw. Betreuer vorgegeben werden.

In dem hier gezeigten Verlauf ist nach der Exsufflation 305 eine Pause 306 vorgesehen. Das bietet dem Patienten eine große Erleichterung, da die Hustenvorgänge eine erhebliche körperliche Anstrengung verlangen. Der hier gezeigte Druckverlauf weist während der Pause 306 einen leichten Überdruck bzw. einen positiven Therapiedruck auf. Das Ausatmen gegen einen solchen leichten, gezielten Überdruck ist atemtherapeutisch besonders sinnvoll. Der Überdruck kann beispielsweise als ein konstanter positiver Druck (CPAP) ausgebildet sein.

Beispielsweise liegt der Druck zwischen 4 und 30 mbar. Hingegen kann für die Exsufflation und/oder Insufflation ein Druck im Bereich von etwa +/-70 mbar oder auch höher eingestellt werden. In der Pause ergeben sich im Rahmen von Ein- und Ausatmung typischerweise erheblich kleinere Flows im Vergleich zur In- oder Exsufflation.

In der Pause kann auch eine Beatmung vorgesehen sein. Dann ist insbesondere die Beatmungseinrichtung 9 aktiv. Beispielsweise ist dann für die Beatmung bzw. Inspiration ein Druck bis etwa 50 mbar und insbesondere zwischen 10-35 mbar vorgesehen.

Der Abfall des Drucks 301 bei dem Übergang von Insufflation zu Exsufflation erfolgt hier vorzugsweise durch ein entsprechend zügiges Umschalten der Ventileinheit 7. Die Drehzahl des Lüfters 6 für die Exsufflation wird vorzugsweise bereits vor dem Schaltvorgang der Ventileinheit 7 entsprechend angepasst. Erfindungsgemäß ist dies aber nicht notwendig.

Der Anstieg des Drucks 301 von der Exsufflation zur nächsten Insufflation oder nach einer Pause hin zur folgenden Insufflation erfolgt vorzugsweise weniger zügig bzw. über einen längeren Zeitraum. Der Druckanstieg kann neben der Änderung der Ventilstellung durch ein entsprechend behutsames Hochfahren des entsprechenden Lüfters 5, 6 erfolgen.

Auch die Erhöhung des Drucks 301 im Vorlauf für die Pause 306 erfolgt hier durch eine entsprechend langsame Drehzahlerhöhung des Lüfters 5.

Die Figur 11 zeigt ein Beispiel für ein Hustenmanöver mit einer anschließenden Pause 306. Dazu wurde in der oberen Grafik der Druck 301 gegen die Zeit 302 aufgetragen. In der mittleren Grafik wurde eine Drehzahl 307 des ersten Lüfters 5 beispielhaft und stark idealisiert gegen die Zeit 302 aufgetragen. In der unteren Grafik wurde eine Drehzahl 307 des zweiten Lüfters 6 beispielhaft und stark idealisiert gegen die Zeit 302 aufgetragen. Die vertikal verlaufenden, gestrichelten Linien deuten hierbei stark schematisiert ein Umschalten der Ventilstellung an.

Zu Beginn des Manövers wird die Ventileinheit 7 in die Ventilstellung für die Insufflation gebracht. Gemäß der in der Figur 1 gezeigten Ausführung wird dazu die erste Ventilstellung 17 eingenommen, sodass der erste Lüfter 5 zugeschaltet wird. Dann wird die Drehzahl 307 des ersten Lüfters 5 über eine definierte Zeit langsam erhöht. Entsprechend erhöht sich der Druck 301. Nach Erreichen des für die Insufflation erforderlichen Drucks 301 wird die Drehzahl 307 gehalten.

Nach einer bestimmten Zeit erfolgt der Wechsel von der Insufflation 304 zu Exsufflation 305. Für ein wirksames Auslösen des Hustenreizes bzw. für eine besonders wirksame Unterstützung der Sekretabfuhr erfolgt der Wechsel hier besonders kurzfristig. Dazu wird die Ventileinheit 7 in die zweite Ventilstellung 27 geschaltet. Der Druck 301 fällt über einen sehr kurzen Zeitraum entsprechend ab. Der für die Exsufflation 305 notwendige negative Druck 101 wird erreicht. Um den Druckübergang besonders kurzfristig ermöglichen zu können, wurde die Drehzahl 307 des zweiten Lüfters 6 bereits vor dem Zuschalten auf das erforderliche Maß erhöht. Der Druck 301 bzw. die Drehzahl 107 für die Exsufflation 305 werden nun für eine vorbestimmte Zeit 102 gehalten. Der zweite Lüfter 6 erreicht seinen Ziel-Betriebsbereich also vor der Umschaltung in die Exsufflationsphase.

Anschließend erfolgt wieder ein Umschalten der Ventileinheit 7 in die erste Ventilstellung 17. Nun ist also wieder der erste Lüfter 5 zugeschaltet. Nach dem Zuschalten wird die Drehzahl 307 des ersten Lüfters 5 soweit erhöht, dass ein entsprechend leichter und für die Beatmung während der Pause 306 geeigneter Überdruck vorliegt. Der erste Lüfter 5 beschleunigt also während des Druckaufbaus bzw. zur Erzeugung des Druckverlaufs. Der zweite Lüfter 6 ist nun nicht mehr zugeschaltet, sodass dessen Drehzahl 107 entsprechend abgesenkt werden kann.

Nach dem Ende der Pause 306 kann wieder eine Erhöhung der Drehzahl 107 des ersten Lüfters 5 erfolgen, um den für die Insufflation 304 benötigten Druck 301 zu erreichen. Das Hustenmanöver kann nun von Neuem beginnen.

Insgesamt bietet die hier vorgestellte Erfindung den Vorteil, dass eine besonders patientenfreundliche und zugleich wirksame Hustenmaschine bereitgestellt wird. Zudem bietet die Erfindung den Vorteil, dass auch eine erheblich verbesserte Beatmung möglich ist. So kann während der Beatmung z. B. eine besonders sanfte Unterstützung bei der Sekretabfuhr erfolgen, in dem der Patient in einer Ausatemphase mit einem negativen Therapiedruck unterstützt wird. Besonders vorteilhaft kann die Erfindung dabei mit einem Zweischlauchsystem eingesetzt werden.

Ein weiterer Vorteil ist, dass die Beatmung alleine oder auch in Kombination mit einer Husten- bzw. Sekrettherapie erfolgen kann. Beispielsweise erfolgt während einer Husten- bzw. Sekrettherapie eine Pause, in welcher ein positiver Therapiedruck eingesetzt wird, um den Patienten zu entlasten. In der Pause kann auch eine Beatmung des Patienten erfolgen.

**Bezugszeichenliste:**

| | | | |
|---|---|---|---|
| 1 | Atemtherapiegerät | 57 | Proportionalventil |
| 2 | Strömungseinrichtung | 67 | Drehschieberventil |
| 3 | Patientenschnittstelle | 77 | Ventilstellung |
| 4 | Atemluftschnittstelle | 87 | Ventilstellung |
| 5 | Lüfter | 97 | Anschluss |
| 6 | Lüfter | 107 | Antrieb |
| 7 | Ventileinheit | 117 | Ventilkolben |
| 8 | Oszillatoreinrichtung | 200 | Schlaucheinrichtung |
| 9 | Beatmungseinrichtung | 201 | Einatemschlauch |
| 10 | Hustengerät | 202 | Ausatemschlauch |
| 11 | Steuereinrichtung | 203 | Patientenventil |
| 13 | Kupplungseinrichtung | 204 | Patienteninterface |
| 14 | Lufteinlass | 301 | Druck |
| 15 | Ansaugseite | 302 | Zeit |
| 16 | Ansaugseite | 303 | Oszillation |
| 17 | Ventilstellung | 304 | Insufflation |
| 24 | Luftauslass | 305 | Exsufflation |
| 25 | Abgabeseite | 306 | Pause |
| 26 | Abgabeseite | 307 | Drehzahl |
| 27 | Ventilstellung | 701 | Anschluss |
| 34 | Öffnung | 702 | Anschluss |
| 37 | Ventilstellung | 703 | Anschluss |
| 47 | Wegeventil | | |

## Patentansprüche

1. Atemtherapiegerät (1) zur gezielten Unterstützung einer Sekretabfuhr aus den Atemwegen eines Patienten mit wenigstens einer Strömungseinrichtung (2) zum Erzeugen wenigstens einer Atemluftströmung für eine Insufflation in den Patienten und zum Erzeugen wenigstens einer Atemluftströmung für eine Exsufflation aus dem Patienten, wobei die Strömungseinrichtung (2) wenigstens eine Patientenschnittstelle (3) zum Verbinden des Patienten mit dem Atemtherapiegerät (1) und wenigstens eine Atemlüftschnittstelle (4) zum Verbinden des Atemtherapiegeräts (1) mit der Atemluft oder Umgebungsluft umfasst, wobei die Strömungseinrichtung (2) wenigstens zwei Strömungswege aufweist, wobei jeder Strömungsweg wenigstens eine Gasquelle (5, 6) mit jeweils wenigstens einer Ansaugseite (15, 16) und jeweils wenigstens einer Abgabeseite (25, 26) umfasst und wobei wenigstens eine erste Gasquelle (5) mit ihrer Ansaugseite (15) °und wenigstens eine zweite Gasquelle (6) mit ihrer Abgabeseite (26) an wenigstens eine schaltbare Ventileinheit (7) strömungstechnisch gekoppelt ist wobei die Gasquellen als Lüfter (5, 6) ausgebildet sind und, wobei das Atemtherapiegerät (1) eine Steuereinrichtung (11) zur Ansteuerung der Ventileinheit (7) und der Lüfter (5, 6) umfasst,
wobei das Atemtherapiegerät (1) eine Sensoreinrichtung zur Überwachung des Volumendurchfluss und/oder des Drucks der Atemgasströmung umfasst,
wobei die Steuereinrichtung (11) ausgebildet ist, in Abhängigkeit der Sensorsignale die Ventileinheit (7) und/oder die Lüfter (5, 6) in Abhängigkeit der erfassten Sensorsignale einzustellen bzw. zu regeln, **dadurch gekennzeichnet, dass** die Ventileinheit (7) strömungstechnisch zwischen den wenigstens zwei Lüftern (5, 6) und der Atemluftschnittstelle (4) angeordnet ist.

2. Atemtherapiegerät (1) nach Anspruch 1, wobei die Gasquellen (5, 6) als elektronisch betriebene Lüfter (5, 6) ausgebildet sind.

3. Atemtherapiegerät (1) nach Anspruch 2, wobei die Lüfter (5, 6) invers zueinander in den Strömungswegen angeordnet sind, wobei die Strömungswege parallel verlaufen.

4. Atemtherapiegerät (1) nach einem der vorhergehenden Ansprüche, wobei die Ventileinheit (7) dazu geeignet und ausgebildet ist, in wenigstens einer ersten Ventilstellung (17) die Ansaugseite (15) des ersten Lüfters (5) mit der Atemluftschnittstelle (4) oder mit der Patientenschnittstelle (3) zu verbinden und die Abgabeseite (26) des zweiten Lüfters (6) zu sperren.

5. Atemtherapiegerät (1) nach einem der vorhergehenden Ansprüche, wobei die Ventileinheit (7) dazu geeignet und ausgebildet ist, in wenigstens einer zweiten Ventilstellung (27) die Ansaugseite (15) des ersten Lüfters (5) zu sperren und die Abgabeseite (26) des zweiten Lüfters (6) mit der Atemluftschnittstelle (4) oder mit der Patientenschnittstelle (3) zu verbinden.

6. Atemtherapiegerät (1) nach einem der vorhergehenden Ansprüche, wobei die Ventileinheit (7) dazu geeignet und ausgebildet ist, in wenigstens einer dritten Ventilstellung (37) die Ansaugseite (15) des ersten Lüfters (5) zu sperren und die Abgabeseite (26) des zweiten Lüfters (6) zu sperren.

7. Atemtherapiegerät (1) nach einem der vorhergehenden Ansprüche, wobei die Ventileinheit (7) wenigstens ein 3/3-Wegeventil (47) und/oder wenigstens ein Drehschieberventil (67) umfasst.

8. Atemtherapiegerät (1) nach einem der vorhergehenden Ansprüche, wobei die Ventileinheit (7) als ein Proportionalventil (57) ausgebildet ist.

9. Atemtherapiegerät (1) nach einem der vorhergehenden Ansprüche, wobei die Strömungseinrichtung (2) dazu geeignet und ausgebildet ist, wenigstens einen der wenigstens zwei Lüfter (5, 6) auch dann zu betreiben, wenn die Ansaugseite (15, 16) oder Abgabeseite (25, 26) des Lüfters (5, 6) durch die Ventileinheit (7) gesperrt ist.

10. Atemtherapiegerät (1) nach einem der vorhergehenden Ansprüche, wobei die Strömungseinrichtung (2) dazu geeignet und ausgebildet ist, eine geforderte Drehzahl wenigstens eines der wenigstens zwei Lüfter (5, 6) einzustellen, während die Ansaugseite (15, 25) oder Abgabeseite (25, 26) des einzustellenden Lüfters (5, 6) durch die Ventileinheit (7) gesperrt ist.

11. Atemtherapiegerät (1) nach einem der vorhergehenden Ansprüche, umfassend wenigstens eine Oszillatoreinrichtung (8) zum Beaufschlagen der Atemluftströmung für die Insufflation und/oder Exsufflation mit wenigstens einer definierten Schwingung.

12. Atemtherapiegerät (1) nach dem vorhergehenden Anspruch, wobei die Oszillatoreinrichtung (8) dazu geeignet und ausgebildet ist, die Schwingung durch ein wiederholtes Umschalten der Ventileinheit (7) zu erzeugen.

13. Atemtherapiegerät (1) nach einem der beiden vorhergehenden Ansprüche, wobei die Oszillatoreinrichtung (8) dazu geeignet und ausgebildet ist, während der Insufflation und/oder der Exsufflation die Ventileinheit (7) zwischen einer voll geöffneten Ventilstellung (77) und einer teilweise geöffneten Ventilstellung (87) umzuschalten.

14. Atemtherapiegerät (1) nach einem der drei vorhergehenden Ansprüche, wobei die Oszillatoreinrichtung (8) dazu geeignet und ausgebildet ist, während der Insufflation die Ventileinheit (7) zwischen einer wenigstens teilweise geöffneten Ventilstellung (87) für die Insufflation und einer wenigstens teilweise geöffneten Ventilstellung (87) für die Exsufflation umzuschalten und/oder während der Exsufflation die Ventileinheit (7) zwischen einer wenigstens teilweise geöffneten Ventilstellung (87) für die Exsufflation und einer wenigstens teilweise geöffneten Ventilstellung (87) für die Insufflation umzuschalten.

15. Atemtherapiegerät (1) nach einem der vier vorhergehenden Ansprüche, wobei die Oszillatoreinrichtung (8) dazu geeignet und ausgebildet ist, während der Insufflation einen anderen maximalen und/oder minimalen Öffnungsgrad der Ventileinheit (7) als während der Exsufflation einzustellen.

16. Atemtherapiegerät (1) nach einem der fünf vorhergehenden Ansprüche, wobei die Oszillatoreinrichtung (8) dazu geeignet und ausgebildet ist, die Ventileinheit (7) mit einer Frequenz von 0,1 Hz bis 100 Hz umzuschalten.

17. Atemtherapiegerät (1) nach einem der sechs vorhergehenden Ansprüche, wobei die Oszillatoreinrichtung (8) dazu geeignet und ausgebildet ist, für die Insufflation eine andere Frequenz und/oder Amplitude als für die Exsufflation einzustellen.

18. Atemtherapiegerät (1) nach einem der vorhergehenden Ansprüche, wobei die Atemluftschnittstelle (4) wenigstens einen Lufteinlass (14) und wenigstens einen Luftauslass (24) umfasst und wobei der Lufteinlass (14) und der Luftauslass (24) durch eine gemeinsame Öffnung (34) bereitgestellt werden.

19. Atemtherapiegerät (1) nach einem der vorhergehenden Ansprüche oder nach dem Oberbegriff von Anspruch 1, umfassend wenigstens eine Beatmungseinrichtung (9), welche dazu geeignet und ausgebildet ist, mittels der Strömungseinrichtung (2) eine Atemluftströmung zur Beatmung des Patienten zu erzeugen.

## Claims

1. A respiratory therapy device (1) for the targeted assistance of a secretion removal from the airways of a patient, having at least one flow apparatus (2) for generating at least one respiratory airflow for an insufflation into the patient and for generating at least one respiratory airflow for an exsufflation out of the patient, wherein the flow apparatus (2) comprises at least one patient interface (3) for connecting the patient to the respiratory therapy device (1) and at least one respiratory air interface (4) for connecting the respiratory therapy device (1) to respiratory air or ambient air, wherein the flow apparatus (2) has at least two flow paths, wherein each flow path comprises at least one gas source (5, 6), each having at least one intake side (15, 16) and at least one delivery side (25, 26), and wherein at least one first gas source (5) is fluidically coupled with its intake side (15) and at least one second gas source (6) is fluidically coupled with its delivery side (26) to at least one switchable valve unit (7), wherein the gas sources are designed as fans (5, 6) and wherein the respiratory therapy device (1) comprises a control apparatus (11) for actuating the valve unit (7) and the fans (5, 6), wherein the respiratory therapy device (1) comprises a sensor apparatus for monitoring the volume flow rate and/or the pressure of the respiratory gas flow, wherein the control apparatus (11) is designed for setting or respectively regulating the valve unit (7) as a function of the sensor signals and/or for setting or respectively regulating the fans (5, 6) as a function of the captured sensor signals, **characterized in that** the valve unit (7) is fluidically arranged between the at least two fans (5, 6) and the respiratory air interface (4) .

2. The respiratory therapy device (1) according to Claim 1, wherein the gas sources (5, 6) are designed as electronically operated fans (5, 6).

3. The respiratory therapy device (1) according to Claim 2, wherein the fans (5, 6) are arranged inversely in relation to one another in the flow paths, wherein the flow paths extend in parallel.

4. The respiratory therapy device (1) according to any one of the preceding claims, wherein the valve unit (7) is suitable and designed for connecting the intake side (15) of the first fan (5) to the respiratory air interface (4) or to the patient interface (3) and blocking the delivery side (26) of the second fan (6) in at least one first valve position (17).

5. The respiratory therapy device (1) according to any one of the preceding claims, wherein the valve unit (7) is suitable and designed for blocking the intake side (15) of the first fan (5) and connecting the delivery side (26) of the second fan (6) to the respiratory air interface (4) or to the patient interface (3) in at least one second valve position (27).

6. The respiratory therapy device (1) according to any one of the preceding claims, wherein the valve unit (7) is suitable and designed for blocking the intake side (15) of the first fan (5) and blocking the delivery side (26) of the second fan (6) in at least one third valve position (37) .

7. The respiratory therapy device (1) according to any one of the preceding claims, wherein the valve unit (7) comprises at least one 3/3-directional valve (47) and/or at least one rotary slide valve (67).

8. The respiratory therapy device (1) according to any one of the preceding claims, wherein the valve unit (7) is designed as a proportional valve (57).

9. The respiratory therapy device (1) according to any one of the preceding claims, wherein the flow apparatus (2) is suitable and designed for operating at least one of the at least two fans (5, 6) even if the intake side (15, 16) or delivery side (25, 26) of the fan (5, 6) is blocked by the valve unit (7).

10. The respiratory therapy device (1) according to any one of the preceding claims, wherein the flow apparatus (2) is suitable and designed for setting a requested speed of at least one of the at least two fans (5, 6) while the intake side (15, 25) or delivery side (25, 26) of the fan (5, 6) to be set is blocked by the valve unit (7).

11. The respiratory therapy device (1) according to any one of the preceding claims, comprising at least one oscillator apparatus (8) for applying at least one defined oscillation to the respiratory airflow for the insufflation and/or exsufflation.

12. The respiratory therapy device (1) according to the preceding claim, wherein the oscillator apparatus (8) is suitable and designed for generating the oscillation by repeated switching over of the valve unit (7).

13. The respiratory therapy device (1) according to any one of the two preceding claims, wherein the oscillator apparatus (8) is suitable and designed for switching over the valve unit (7) between a fully open valve position (77) and a partially open valve position (87) during insufflation and/or exsufflation.

14. The respiratory therapy device (1) according to any one of the three preceding claims, wherein the oscillator apparatus (8) is suitable and designed for switching over the valve unit (7) between an at least partially open valve position (87) for insufflation and an at least partially open valve position (87) for exsufflation during insufflation and/or for switching over the valve unit (7) between an at least partially open valve position (87) for exsufflation and an at least partially open valve position (87) for insufflation during exsufflation.

15. The respiratory therapy device (1) according to any one of the four preceding claims, wherein the oscillator apparatus (8) is suitable and designed for setting a different maximum and/or minimum degree of opening of the valve unit (7) during insufflation than during exsufflation.

16. The respiratory therapy device (1) according to any one of the five preceding claims, wherein the oscillator apparatus (8) is suitable and designed for switching over the valve unit (7) at a frequency of 0.1 Hz to 100 Hz.

17. The respiratory therapy device (1) according to any one of the six preceding claims, wherein the oscillator apparatus (8) is suitable and designed for setting a different frequency and/or amplitude for insufflation than for exsufflation.

18. The respiratory therapy device (1) according to any one of the preceding claims, wherein the respiratory air interface (4) comprises at least one air inlet (14) and at least one air outlet (24), and wherein the air inlet (14) and the air outlet (24) are provided by a common opening (34) .

19. The respiratory therapy device (1) according to any one of the preceding claims or according to the preamble of Claim 1, comprising at least one breathing apparatus (9) which is suitable and designed for generating a respiratory airflow for the ventilation of the patient by means of the flow apparatus (2).

## Revendications

1. Appareil de thérapie respiratoire (1) destiné à l'assistance ciblée d'une évacuation de sécrétions hors des voies respiratoires d'un patient avec au moins un dispositif d'écoulement (2) servant à générer au moins un flux d'air respiratoire à insuffler à un patient et au moins un flux d'air respiratoire à exsuffler d'un patient, dans lequel le dispositif d'écoulement (2) comprend au moins une interface patient (3) servant à relier le patient à l'appareil de thérapie respiratoire (1) et au moins une interface d'air respiratoire (4) servant à relier l'appareil de thérapie respiratoire (1) à l'air respiratoire ou à l'air ambiant, dans lequel le dispositif d'écoulement (2) présente au moins deux voies d'écoulement, dans lequel chaque voie d'écoulement comporte au moins une source de gaz (5, 6) avec respectivement au moins un côté d'aspiration (15, 16) et respectivement au moins un côté de distribution (25, 26) et dans lequel une unité à vanne commutable (7) est couplée fluidiquement à au moins une première source de gaz (5) par son côté d'aspiration (15) et à au moins une seconde source de gaz (6) par son côté de distribution (26), dans lequel les sources de gaz sont constituées sous la forme de ventilateurs (5, 6) et dans lequel l'appareil de thérapie respiratoire (1) comprend un dispositif de commande (11) servant à activer l'unité à vanne (7) et les ventilateurs (5, 6), dans lequel l'appareil de thérapie respiratoire (1) comporte un dispositif de détection servant à surveiller le débit volumique et / ou la pression du flux de gaz respiratoire, dans lequel le dispositif de commande (11) est conçu de manière à ajuster ou à réguler l'unité à vanne (7) en fonction des signaux de capteur et / ou les ventilateurs (5, 6) en fonction des signaux de capteur détectés, **caractérisé en ce que** l'unité à vanne (7) est disposée fluidiquement entre au moins les deux ventilateurs (5, 6) et l'interface d'air respiratoire (4) .

2. Appareil de thérapie respiratoire (1) selon la revendication 1, dans lequel les sources de gaz (5, 6) sont constituées sous la forme de ventilateurs actionnés par voie électronique (5, 6).

3. Appareil de thérapie respiratoire (1) selon la revendication 2, dans lequel les ventilateurs (5, 6) sont disposés inversement l'un par rapport à l'autre dans les voies d'écoulement, dans lequel les voies d'écoulement cheminent parallèlement.

4. Appareil de thérapie respiratoire (1) selon l'une des revendications précédentes, dans lequel l'unité à vanne (7) est appropriée et conçue pour relier le côté d'aspiration (15) du premier ventilateur (5) à l'interface d'air respiratoire (4) ou à l'interface patient (3) et pour bloquer le côté de distribution (26) du second ventilateur (6) dans au moins une première position de vanne (17).

5. Appareil de thérapie respiratoire (1) selon l'une des revendications précédentes, dans lequel l'unité à vanne (7) est appropriée et conçue pour bloquer le côté d'aspiration (15) du premier ventilateur (5) et pour relier le côté de "distribution (26) du second ventilateur (6) à l'interface d'air respiratoire (4) ou à l'interface patient (3) dans au moins une deuxième position de vanne (27) .

6. Appareil de thérapie respiratoire (1) selon l'une des revendications précédentes, dans lequel l'unité à vanne (7) est appropriée et conçue pour bloquer le côté d'aspiration (15) du premier ventilateur (5) et pour bloquer le côté de distribution (26) du second ventilateur (6) dans au moins une troisième position de vanne (37).

7. Appareil de thérapie respiratoire (1) selon l'une des revendications précédentes, dans lequel l'unité à vanne (7) comprend au moins une vanne à 3/3 voies (47) et / ou au moins une vanne rotative (67).

8. Appareil de thérapie respiratoire (1) selon l'une des revendications précédentes, dans lequel l'unité à vanne (7) est constituée sous la forme d'une vanne proportionnelle (57).

9. Appareil de thérapie respiratoire (1) selon l'une des revendications précédentes, dans lequel le dispositif d'écoulement (2) est approprié et conçu pour faire fonctionner au moins l'un des deux ventilateurs (5, 6) au moins, même si le côté d'aspiration (15, 16) ou le côté de distribution (25, 26) du ventilateur (5, 6) est bloqué par l'unité à vanne (7).

10. Appareil de thérapie respiratoire (1) selon l'une des revendications précédentes, dans lequel le dispositif d'écoulement (2) est approprié et conçu pour ajuster une vitesse de rotation requise d'au moins l'un des deux ventilateurs (5, 6) au moins, pendant que le côté d'aspiration (15, 25) ou le côté de distribution (25, 26) du ventilateur à ajuster (5, 6) est bloqué par l'unité à vanne (7).

11. Appareil de thérapie respiratoire (1) selon l'une des revendications précédentes, comprenant au. moins un dispositif oscillateur (8) servant à appliquer au moins une oscillation définie au flux d'air respiratoire à insuffler et / ou à exsuffler.

12. Appareil de thérapie respiratoire (1) selon la revendication précédente, dans lequel le dispositif oscillateur (8) est approprié et conçu pour générer l'oscillation moyennant une commutation répétée de l'unité à vanne (7).

13. Appareil de thérapie respiratoire (1) selon l'une des deux revendications précédentes, dans lequel le dispositif oscillateur (8) est approprié et conçu pour commuter l'unité à vanne (7) entre une position de vanne entièrement ouverte (77) et une position de vanne partiellement ouverte (87) durant l'insufflation et / ou l'exsufflation.

14. Appareil de thérapie respiratoire (1) selon l'une des trois revendications précédentes, dans lequel le dispositif oscillateur (8) est approprié et conçu pour commuter l'unité à vanne (7) durant l'insufflation entre une position de vanne au moins partiellement ouverte (87) pour l'insufflation et une position de vanne au moins partiellement ouverte (87) pour l'exsufflation et / ou pour commuter l'unité à vanne (7) durant l'exsufflation entre une position de vanne au moins partiellement ouverte (87) pour l'exsufflation et une position de vanne au moins partiellement ouverte (87) pour l'insufflation.

15. Appareil de thérapie respiratoire (1) selon l'une des quatre revendications précédentes, dans lequel le dispositif oscillateur (8) est approprié et conçu pour ajuster durant l'insufflation un autre degré d'ouverture maximal et / ou minimal de l'unité à vanne (7) que durant l'exsufflation.

16. Appareil de thérapie respiratoire (1) selon l'une des cinq revendications précédentes, dans lequel le dispositif oscillateur (8) est approprié et conçu pour commuter l'unité à vanne (7) à une fréquence de 0,1 Hz à 100 Hz.

17. Appareil de thérapie respiratoire (1) selon l'une des six revendications précédentes, dans lequel le dispositif oscillateur (8) est approprié et conçu pour ajuster pour l'insufflation une autre fréquence et / ou amplitude que pour l'exsufflation.

18. Appareil de thérapie respiratoire (1) selon l'une des revendications précédentes, dans lequel l'interface d'air respiratoire (4) comprend au moins une entrée d'air (14) et au moins une sortie d'air (24) et dans lequel l'entrée d'air (14) et la sortie d'air (24) sont fournies par une ouverture commune (34).

19. Appareil de thérapie respiratoire (1) selon l'une des revendications précédentes ou selon le préambule de la revendication 1, comprenant au moins un dispositif de ventilation (9), lequel est approprié et conçu pour générer un flux d'air respiratoire destiné à la ventilation d'un patient au moyen du dispositif d'écoulement (2).
